# FASCICULE DE BREVET EUROPEEN

(11) **EP 1 511 722 B1**
(45) Date de publication et mention de la délivrance du brevet: **03.10.2007**
(21) Numéro de dépôt: 03755202.3
(22) Date de dépôt: 26.05.2003
(51) Int. Cl.: C07C 311/08, C07C 317/22, A61K 31/18, A61K 31/195

(54) **DERIVES DE PHENYL-CYCLOHEXYL-PROPANOLAMINE, LEUR PREPARATION ET LEUR APPLICATION EN THERAPEUTIQUE**
PHENYLCYCLOHEXYLPROPANOLAMINDERIVATE, DEREN HERSTELLUNG UND THERAPEUTSCHE ANWENDUNG
PHENYLCYCLOHEXYLPROPANOLAMINE DERIVATIVES, PREPARATION AND THERAPEUTIC APPLICATION THEREOF

(30) Priorité: 29.05.2002 FR 0206561
(43) Date de publication de la demande: 09.03.2005
(73) Titulaire: Sanofi-Aventis, 75013 Paris (FR)
(72) Inventeur: BOVY, Philippe, R., F-78750 Mareil Marly (FR); CECCHI, Roberto, I-26900 Lodi (IT); CROCI, Tiziano, I-20155 Milan (IT); VENIER, Olivier, 92500 Rueil Malmaison (FR)
(74) Mandataire: Romanowski, Caroline
(86) Numéro de dépôt international: PCT/FR2003/001580
(87) Numéro de publication internationale: WO 2003/099772

(56) Documents cités:
- EP-A- 0 345 056
- WO-A-02/44139
- WO-A-99/65895
- US-A- 4 004 028

## Description

La présente invention se rapporte à des dérivés de phényl-cyclohexylpropanolamine, à leur préparation et à leur application en thérapeutique.

WO02/44139 divulgue des composés de formula : 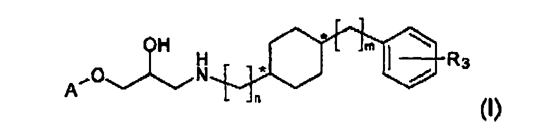 dans laquelle notamment
A peut être un groupe de formule (a) 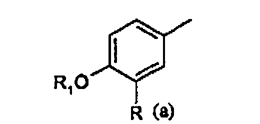 où
R peut représenter un atome d'hydrogène ou d'halogène, un groupe -S(O)₂(C₁-C₄)alkyle, un groupe -NHSO₂(C₁-C₄)alkyle, un groupe -SO₂NH(C₁-C₄)alkyle ou un groupe -NHSO₂phényle ledit phényle pouvant être substitué par un atome d'halogène, par un groupe (C₁-C₄)alkyle ou par un groupe (C₁-C₄)alcoxy ;
R₁ peut représenter un atome d'hydrogène ou un groupe (C₁-C₄)alkyle, un groupe -CO(C₁-C₄)alkyle, un groupe phényl-(C₁-C₄)alkyle ou un groupe -COphényle, ledit phényle pouvant être substitué par un atome d'halogène ou par un groupe (C₁-C₄)alcoxy;
n et m peuvent être égal à 0; z est 0, 1 ou 2 ; R₃ peut représenter un groupe (C₁-C₆)alkyle en tant qu'antagonistes des récepteurs beta-3 adrénergiques.
WO99/65895 divulgue des composés de formule : 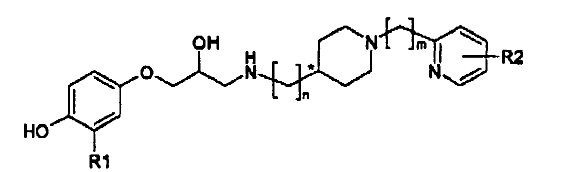 en tant qu'antagonistes des récepteurs beta-3 adrénergiques.
US4,004,028 décrit des dérivés des phénoxypropanolamines de formule 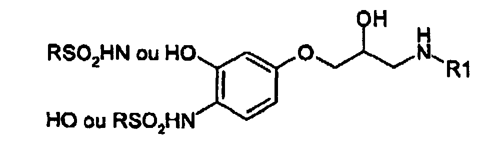 en tant que stimulants des récepteurs beta adrénergiques.
EP 0345056 présente des dérivés de propanolamine de formule Ar-O-CH₂ CH(OH)CH₂NHZ ayant une activité selective sur 5-HT1 et un effet minimal sur les récepteurs beta.

La présente invention a pour objet des composés répondant à la formule (I) 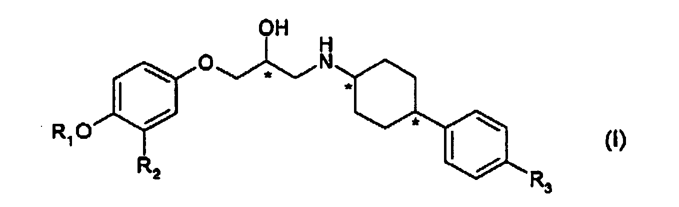 dans laquelle :
**R₁** représente un atome d'hydrogène ou un groupe (C1-C4)alkyle, un groupe -CO(C1-C4)alkyle, un groupe (C1-C4)alkyl-phényle ou un groupe -CO-phényle, ledit phényle étant éventuellement substitué par un à trois groupes choisis indépendamment les uns des autres parmi les atomes d'halogène, les groupes (C1-G4)alkyles et (C1-C4)alcoxy ;
**R₂** est choisi parmi l'un des groupes suivants :
   - un atome d'hydrogène,
   - un atome d'halogène,
   - un groupe -S(O)_{z}(C1-C4)alkyle, où z est égal à 0, 1 ou 2,
   - un groupe -NHSO₂(C1-C4)alkyle,
   - un groupe -NHSO₂-phényle, ou
   - un groupe -NHSO₂-(C1-C4)alkyl-phényle.
   où ledit phényle est éventuellement substitué par un à trois groupes choisis indépendamment les uns des autres parmi les atomes d'halogène, les groupes (C1-C4)alkyles et (C1-C4)alcoxy ; et
R₃ est choisi parmi l'un des groupes suivants :
   - un groupe -X-R₄, dans lequel X représente une liaison, un atome d'oxygène ou un groupe -CH₂- et R₄ représente un atome d'hydrogène ou un groupe de formule -CR₅R₆-COOR₇, où R₅, R₆ et R₇ représentent indépendamment les uns des autres un atome d'hydrogène ou un groupe (C1-C4)alkyle, R₄ ne représentant toutefois pas un atome d'hydrogène lorsque X représente une liaison,
   - un groupe phényle éventuellement substitué par un à trois groupes choisis indépendamment les uns des autres parmi les atomes d'halogène, les groupes (C1-C4)alkyles et (C1-C4)alcoxy, ou fusionné avec un groupe dioxolane (de façon à former un groupe benzodioxolane), ou
   - un groupe -CO-NR₈R₉, dans lequel R₈ représente un atome d'hydrogène, un groupe (C1-C4)alkyle ou un groupe (C1-C4)alkyl-(C1-C4)alcoxy et R₉ est choisi parmi l'un des groupes suivants :
      - un groupe (C1-C4)alkyl-(C1-C4)alcoxy,
      - un groupe de formule -(CH₂)ₙ-A, où n est égal à 0, 1, 2, 3, ou 4 et où A représente un groupe indolyle, fluorène ou phényle, où le groupe phényle est substitué par un à trois groupes choisis indépendamment les uns des autres parmi les atomes d'halogène et les groupes hydroxy ou (C1-C4)alkyles,
      - un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
      - un groupe de formule -CH(R₁₀)-(CH₂)ₙ COOR₁₁, où n est égal à 0, 1, 2 ou 3, R₁₁ représente un atome d'hydrogène ou un groupe (C1-C4)alkyle, et R₁₀ représente :
         - un atome d'hydrogène,
         - un groupe (C1-C4)alkyle,
         - un groupe -COOR₁₂, où R₁₂ représente un atome d'hydrogène ou un groupe (C1-C4)alkyle, ou
         - un groupe -CH₂-phényle, où le groupe phényle est éventuellement substitué par un à trois groupes choisis indépendamment les uns des autres parmi les atomes d'halogène et les groupes hydroxy ou (C1-C4)alkyles.

Les composés de formule (I) peuvent comporter un ou plusieurs atomes de carbone asymétriques. Par ailleurs, le groupe cyclohexyle de ces composés présente une asymétrie géométrique. Les signes * dans la formule (I) ci-dessus désignent les carbones qui peuvent donner lieu à différentes configurations géométriques.

Les composés de formule (I) peuvent donc exister sous forme d'énantiomères ou de diastéréoisomères. Ces énantiomères, diastéréoisomères, ainsi que leurs mélanges, y compris les mélanges racémiques, font partie de l'invention.

On préfère notamment les composés de formule (1) selon l'invention qui présentent la configuration suivante :

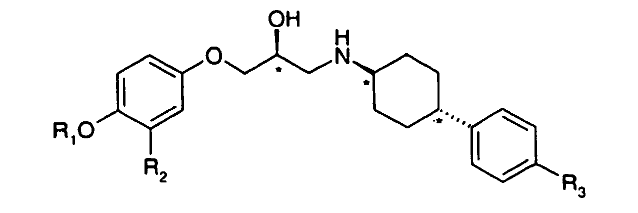

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par:
- un atome d'halogène : un fluor, un chlore, un brome ou un iode ;
- un groupe (C1-C4)alkyle : un groupe aliphatique saturé linéaire ou ramifié comprenant de 1 à 4 atomes de carbone (étant bien entendu qu'un tel groupe ne peut être que linéaire lorsqu'il comprend moins de 3 atomes de carbone, et qu'un tel groupe peut être linéaire ou ramifié lorsqu'il comprend 3 ou 4 atomes de carbone). A titre d'exemples, on peut citer les groupes méthyle, éthyle, propyle, isopropyle, butyle, isobutyle, etc ;
- un groupe (C1-C4)alcoxy : un radical -O-(C1-C4)alkyle, où le groupe (C1-C4)alkyle est tel que précédemment défini ;
- un groupe (C1-C4)alkyl-(C1-C4)alcoxy : un radical de formule (C1-C4)alkyl-O-(C1-C4)alkyle, où le groupe (C1-C4)alkyle est tel que précédemment défini ; et
- un groupe (C1-C4)alkyl-phényle : un groupe de formule -(CH₂)ₓ-phényle, où x est compris entre 1 et 4.

Parmi les composé de formule (I) objets de l'invention, on peut citer les composés préférés dans lesquels R₁ représente un atome d'hydrogène et/ou R₂ représente un groupe -SO₂(C₁-C₄)alkyle (tel qu'un groupe -SO₂CH₃) ou -NHSO₂(C₁-C₄)alkyle (tel qu'un groupe -NHSO₂CH₃).

On préfère également, parmi les composés de formule (I) objets de l'invention, ceux dans lesquels
R₁ représente un atome d'hydrogène ;
et/ou R₂ représente un groupe -SO₂(C1-C4)alkyle (tel qu'un groupe -SO₂CH₃) ou -NHSO_{z}(C1-C4)alkyle (tel qu'un groupe -NHSO₂CH₃) ;
et/ou R₃ est choisi parmi l'un des groupes suivants :
   - un groupe -X-R₄, dans lequel X représente une liaison, un atome d'oxygène ou un groupe -CH₂- et R₄ représente un atome d'hydrogène ou un groupe de formule -CR₅R₆-COOR₇, où R₅, R₆ et R₇ sont tels que définis ci-dessus, R₄ ne représentant toutefois pas un atome d'hydrogène lorsque X représente une liaison ou un groupe -CH₂.
   - un groupe phényle éventuellement substitué par un à trois groupes choisis indépendamment les uns des autres parmi les atomes d'halogène, les groupes (C1-C4)alkyles et (C1-C4)alcoxy, ou fusionné avec un groupe dioxolane, ou
   - un groupe -CO-NR₈R₉, dans lequel R₈ est tel que défini ci-dessus et R₉ est choisi parmi l'un des groupes suivants :
      - un groupe (C1-C4)alkyl-(C1-C4)alcoxy,
      - un groupe de formule -(CH₂)ₙ-A, où n est égal à 0, 1, 2, 3 ou 4 et où A représente un groupe indolyle, un groupe fluorène ou un groupe phényle substitué par un à trois groupes choisis indépendamment parmi les atomes d'halogène et les groupes hydroxy ou (C1-C4)alkyles,
      - un groupe -NH-phényle où le groupe phényle est éventuellement substitué par un à trois groupes choisis indépendamment les uns des autres parmi les atomes d'halogène et les groupes hydroxy ou (C1-C4)alkyles, ou
      - un groupe de formule -CH(R₁₀)-(CH₂)ₙ-COOR₁₁, où n est égal à 0, 1, 2 ou 3, R₁₁ représente un atome d'hydrogène ou un groupe (C1-C4)alkyle, et R₁₀ représente :
         - un atome d'hydrogène,
         - un groupe (C1-C4)alkyle,
         - un groupe -COOR₁₂, où R₁₂ représente un atome d'hydrogène ou un groupe (C1-C4)alkyle, ou
         - un groupe -CH₂-phényle, où le groupe phényle est éventuellement substitué par un à trois groupes choisis indépendamment les uns des autres parmi les atomes d'halogène et les groupes hydroxy ou (C1-C4)alkyles.

On peut encore citer les composés de formule (I) préférés dans lesquels :
R₁ représente un atome d'hydrogène ;
et/ou R₂ représente un groupe -SO₂(C₁-C₄)alkyle (tel qu'un groupe -SO₂CH₃) ou -NHSO₂(C1-C4)alkyle (tel qu'un groupe -NHSO₂CH₃) ;
et/ou R₃ représente un groupe -CO-NHR₉, dans lequel R₉ est choisi parmi l'un des groupes suivants :
   - un groupe de formule -(CH₂)ₙ-A, où n est égal à 0, 1, 2, 3 ou 4 et où A représente un groupe indolyle, un groupe fluorène ou un groupe phényle substitué par un à trois groupes choisis indépendamment parmi les atomes d'halogène et les groupes hydroxy ou (C1-C4)alkyles
   - un groupe -NH-phényle où le groupe phényle est éventuellement substitué par un à trois groupes choisis indépendamment les uns des autres parmi les atomes d'halogène et les groupes hydroxy ou (C1-C4)alkyles, ou
   - un groupe de formule -CH(R₁₀)-(CH₂)ₙ-COOR₁₁, où n est égal à 0, 1, 2 ou 3, R₁₁ représente un atome d'hydrogène ou un groupe (C1-C4)alkyle, et R₁₀ représente :
      - un atome d'hydrogène,
      - un groupe (C1-C4)alkyle,
      - un groupe -COOR₁₂, où R₁₂ représente un atome d'hydrogène ou un groupe (C1-C4)alkyle, ou
      - un groupe -CH₂-phényle, où le groupe phényle est éventuellement substitué par un à trois groupes choisis indépendamment les uns des autres parmi les atomes d'halogène et les groupes hydroxy ou (C1-C4)alkyles.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective groups in Organic Synthesis », Green et al., 2nd Edition (John Wiley & Sons, Inc., New York).

Conformément à l'invention, on peut préparer les composés de formule générale (I), où R₃ est différent d'un groupe -CO-NR₈R₉ (c'est-à-dire où R₃ représente un groupe -X-R₄ ou un groupe phényle éventuellement substitué ou fusionné avec un ou plusieurs autres groupes, tel que défini ci-avant), selon le procédé décrit dans le schéma 1.

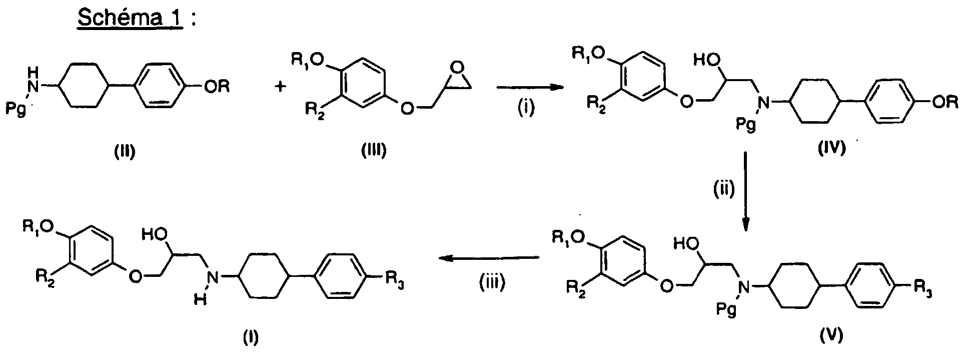

Selon le schéma 1, on fait réagir, dans une étape (i), le composé de formule (II), où Pg représente un groupe protecteur et R un groupe électrophile, avec l'époxyde de formule (III), où R₁ et R₂ sont tels que précédemment définis.

Les composés de formule (III) sont connus dans la littérature (par exemple dans la demande de brevet publiée sous le numéro WO 02/44139) ou bien peuvent être préparés par des procédés analogues aux procédés qui y sont décrits. Dans le cas où R₂ peut réagir au cours de cette étape (i) ou des étapes ultérieures, on le protège préalablement au moyen des groupes protecteurs bien connus de l'Homme du métier. Par ailleurs, lorsque R₁ représente un atome d'hydrogène, il est préférable de protéger le groupe fonctionnel hydroxy par un groupe protecteur afin d'augmenter le rendement de la réaction. A cet effet, on peut utiliser les groupes protecteurs usuels pour les groupes phénols, tels que le méthoxyéthoxyméthyle (MEM), le triméthylsilyl-éthoxyméthyle (SEM), le benzyle éventuellement substitué, ou le benzoyle.

Quant à l'amine du composé (II), elle est partiellement protégée à l'aide d'un groupe protecteur Pg, tel qu'un groupe benzyle éventuellement substitué (par exemple un groupe para-méthoxybenzyle) ou un groupe méthoxyéthoxyméthyle (MEM). On préfère ici utiliser des groupes protecteurs qui ne protègent que partiellement la réactivité de la fonction amine, c'est-à-dire qui n'altèrent pas son caractère nucléophile. Lors de l'étape (i), l'amine primaire partiellement protégée, dans le composé (II), ne peut réagir qu'avec une seule molécule de l'époxyde (III), et non avec deux molécules, évitant ainsi la formation de sous-produits réactionnels.L'étape (i) conduit à l'amino-alcool de formule (IV). Cette étape est par exemple réalisée dans un solvant organique, tel qu'un alcool inférieur comme le méthanol, l'éthanol, l'isopropanol ou le tert-butanol, ou encore dans le diméthylsulfoxyde, dans un éther linéaire ou cyclique, dans un amide comme le diméthylformamide ou le diméthylacétamide, ou encore dans un mélange de ces solvants, en utilisant de préférence des quantités au moins équimolaires des réactifs. La température de la réaction est avantageusement comprise entre la température ambiante et la température de reflux du solvant choisi.

Dans une étape (ii), on transforme le groupe OR en fonction R₃, R₃ étant tel que défini précédemment, mais étant différent d'un groupe -CO-NR₈R₉. Par exemple, dans le cas où le groupe OR représente une fonction triflate, on peut réaliser un couplage avec un dérivé arylboronique ou arylstanneux avec une catalyse par un métal de transition tel que le palladium, en présence d'une phosphine, dans un solvant tel que le toluène, le tétrahydrofurane, le DME ou le diméthylformamide, et si nécessaire en présence d'une base (par exemple le carbonate de sodium) et d'eau ; on obtient alors un composé (V) où R₃ est un groupe aryle, par exemple un groupe phényle éventuellement substitué ou fusionné avec un ou plusieurs autres groupes tels que définis dans la formule (I) des composés selon l'invention.

Dans le cas où OR représente un groupe -O-CR₅R₆-COOR₇, où R₅ et R₆ sont tels que définis dans la formule (I) et R₇ représente un groupe (C1-C4)alkyle, on peut réaliser lors de l'étape (ii) l'hydrolyse de la fonction ester en acide, pour obtenir un composé (V) dans lequel R₃= -O-CR₅R₆-COOH, par traitement par une base, par exemple la soude dans un solvant ou un mélange de solvants, tel qu'un mélange éthanol/eau.

Il est bien entendu que, lorsque le groupe OR dans le composé (II) de départ représente déjà le groupe R₃ souhaité, alors l'étape (ii) n'a pas lieu d'être. Par exemple, lorsque OR représente un groupe hydroxyle, alors on obtient directement le composé (V) souhaité dans lequel R₃ = OH, par réaction entre les composés (II) et (III).

Les composés de formule (I) sont finalement obtenus, dans une étape (iii), après élimination des groupes protecteurs au moyen des techniques connues de l'Homme du métier. En particulier, lorsque les groupes protecteurs sont des groupes benzyles, on réalise la déprotection au moyen d'hydrogène en présence de palladium sur charbon, dans un solvant tel que l'éthanol. Cependant, dans le cas où R2 représente un groupe -S(O)(C1-C4)alkyle, on préférera éviter cette étape d'élimination des groupes protecteurs, et on utilisera de préférence, en tant que produit de départ, un composé (II) portant une amine primaire (Pg = H pour les composés (II), (IV) et (V) dans le schéma 1).

La préparation des composés de formule (II), utilisés comme produits de départ dans le procédé selon le schéma 1, peut être réalisée selon le procédé présenté dans le schéma 2.

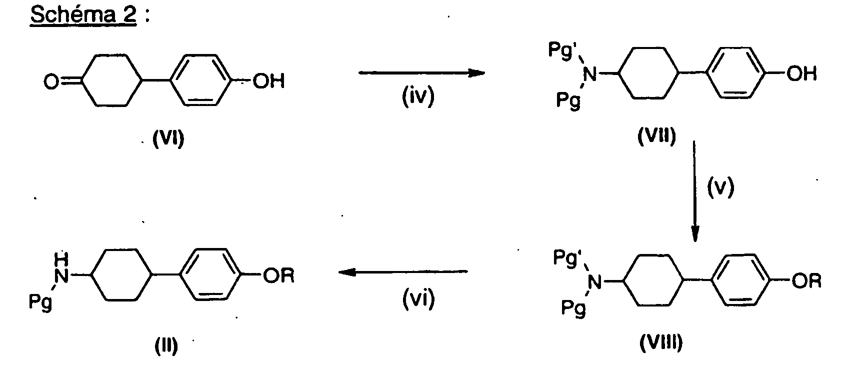

Dans le schéma 2, on transforme la fonction cétone du composé de formule (VI), dans une étape (iv), en une fonction amine selon les méthodes bien connues de l'Homme du métier (par exemple par amination réductrice). Cette amine se trouve protégée à l'aide de deux groupes protecteurs Pg et Pg'. Pg et Pg' sont de natures chimiques différentes, en particulier pour leur mode de déprotection (ce qui permet ensuite une déprotection sélective de l'un ou l'autre des groupes protecteurs). Par exemple, Pg peut être un groupe benzyle et Pg' une fonction carbamate. Pg et Pg' sont introduits selon les méthodes bien connues de l'Homme du métier (par exemple par addition d'un anhydride ou d'un chlorure d'acide pour l'introduction d'un motif carbamate), et en général après la transformation de la fonction cétone du composé (VI) en fonction amine.

Lors de l'étape (v), on obtient le composé (VIII) en faisant réagir la fonction phénol du composé (VII) avec un électrophile (groupe R), qui peut être un anhydride d'acide, un halogénure d'acide ou un dérivé alkyle halogéné. Quand on fait réagir un halogénure d'acide ou un anhydride d'acide (par exemple l'anhydride trifluorométhanesulfonique), on réalise la réaction dans un solvant tel que le tétrahydrofurane, un éther linéaire, le dichlorométhane ou le toluène et en présence d'une base tel que la triéthylamine, la pyridine ou la diisopropyléthylamine. Dans le cas où l'on fait réagir un dérivé alkyle halogéné (par exemple le bromo acétate d'éthyle), on réalise la réaction dans un solvant tel que le tétrahydrofurane, l'acétone ou le diméthylformamide et en présence d'une base telle que l'hydrure de sodium, le bicarbonate de potassium ou la soude.

Le composé (II) est obtenu, dans la dernière étape (vi), par déprotection sélective du groupe protecteur Pg' de la cyclohexylamine (VIII) selon les méthodes bien connues de l'Homme du métier. Par exemple, dans le cas où Pg est un groupe benzyle et Pg' est un groupe *tert*-butoxy carbonyle, la déprotection est réalisée au moyen d'une solution d'acide trifluororacétique dans le dichlorométhane.

Par ailleurs, on peut préparer les composés de formule générale (I), où R₃ représente un groupe -CO-NR₈R₉ (R₈ et R₉ étant tels que définis précédemment en rapport avec la formule (I) des composés selon l'invention), selon le procédé décrit dans le schéma 3.

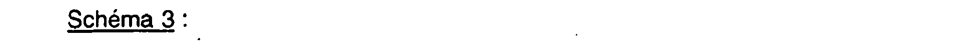

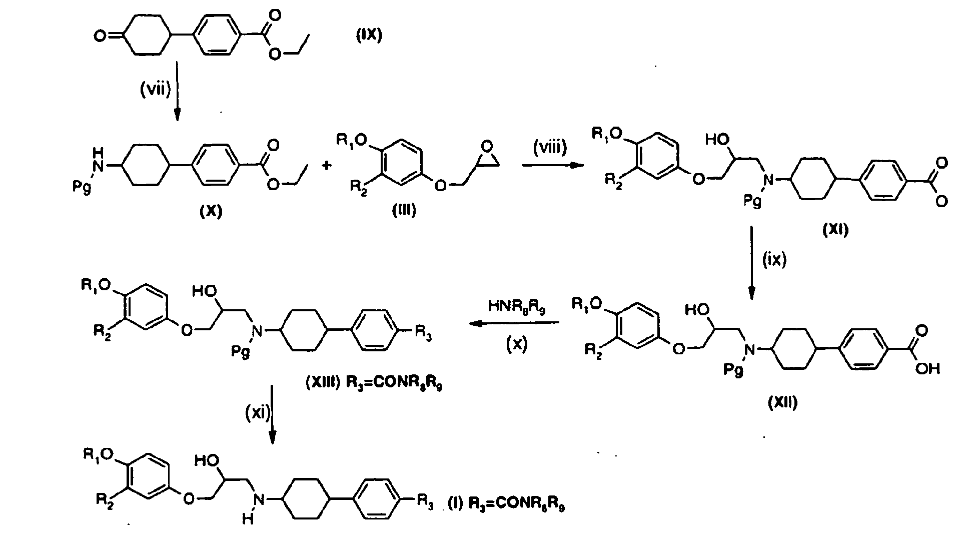

Selon le schéma 3, on transforme la fonction cétone du composé de formule (IX), dans une étape (vii), en un groupe amine selon les méthodes bien connues de l'Homme du métier (par exemple par amination réductrice). L'amine (X) se trouve partiellement protégée à l'aide d'un groupe protecteur Pg, tel qu'un groupe benzyle éventuellement substitué (par exemple un groupe para-méthoxybenzyle) ou un groupe méthoxyéthoxyméthyle (MEM). On préfère ici utiliser des groupes protecteurs qui ne protègent que partiellement la réactivité de la fonction amine, c'est-à-dire qui n'altèrent pas son caractère nucléophile. Cependant, dans le cas où R2 représente un groupe -S(O)(C1-C4)alkyle, on utilisera de préférence un composé (X) portant une amine primaire (Pg = H pour le composé (X) dans le schéma 3).

Dans une étape (viii), on fait réagir le composé de formule (X) avec l'époxyde de formule (III), déjà définis dans le schéma 1 ci-avant. Dans le cas où R2 représente un groupe -S(O)(C1-C4)alkyle, on protègera ensuite l'azote du composé (XI) avec un groupe amino-protecteur, comme le t-butyloxycarbonyl (BOC) (Pg = BOC dans le schéma 3 pour les composés (XI), (XII) et (XIII)), selon les méthodes connues de l'Homme du métier. Dans le cas où R₂ peut réagir au cours de cette étape (viii) ou des étapes ultérieures, on le protège préalablement au moyen des groupes protecteurs bien connus de l'Homme du métier. Par ailleurs, lorsque R₁ représente un atome d'hydrogène, il est préférable de protéger le groupe fonctionnel hydroxy par un groupe protecteur afin d'augmenter le rendement de la réaction, comme explicité précédemment en rapport avec le schéma 1.

Lors de l'étape (viii), l'amine primaire partiellement protégée, dans le composé (X), ne peut réagir qu'avec une seule molécule de l'époxyde (III), et non avec deux molécules, évitant ainsi la formation de sous-produits réactionnels.

L'étape (viii) conduit à l'amino-alcool de formule (XI). Cette étape est par exemple réalisée dans un solvant organique, tel qu'un alcool inférieur comme le méthanol, l'éthanol, l'isopropanol ou le tert-butanol, ou encore dans le diméthylsulfoxyde, dans un éther linéaire ou cyclique, dans un amide comme le diméthylformamide ou le diméthylacétamide, ou encore dans un mélange de ces solvants, en utilisant de préférence des quantités au moins équimolaires des réactifs. La température de la réaction est avantageusement comprise entre la température ambiante et la température de reflux du solvant choisi.

Dans une étape (ix), l'ester éthylique du composé (XI) est hydrolysé en acide (XII) par traitement par une base, par exemple la soude dans un solvant ou un mélange de solvants, tel qu'un mélange éthanol/eau.

L'amide de formule (XIII) est obtenu, dans une étape (x), en faisant réagir l'acide (XII) avec une amine de formule HNR₈R₉, dans laquelle R₈ et R₉ sont tels que définis en rapport avec la formule (I) précédemment décrite, en présence d'un agent de couplage, par exemple le 1-éthyl-3-(3-diméthylaminopropyl)carbodiimide (EDC), l'hexafluorophosphate de benzotriazol-1-yloxy-tris(diméthylamino)phosphonium (BOP) ou le tétrafluoroborate de benzotriazol-1-yloxy-N, N'-tétraméthyluronium (TBTU), et en présence d'un base telle que la triéthylamine ou la pyridine, dans un solvant tel que le dichlorométhane, l'acétonitrile ou le chloroforme. Il est également possible d'activer la fonction acide du composé (XII) sous forme d'un chlorure d'acide ou d'un anhydride carbonique, selon les techniques connues de l'Homme du métier.

Les composés de formule (I) sont finalement obtenus, dans une étape (xi), après élimination des groupes protecteurs au moyen des techniques connues de l'Homme du métier, ou si nécessaire après transformation d'un groupe ester en fonction acide puis élimination des groupes protecteurs. En particulier, lorsque les groupes protecteurs sont des groupes benzyles, on réalise la déprotection au moyen d'hydrogène en présence de palladium sur charbon, dans un solvant tel que l'éthanol.

Dans le cas où le composé (XIII) comprend, en tant que groupe R₉, un groupe ester de formule -CH(R₁₀)-(CH₂)ₙ-COOR₁₁, où R₁₁ représente un groupe (C1-C4)alkyle et R₁₀ est tel que défini précédemment en rapport avec les composés de formule (I) conformes à l'invention, alors l'étape d'élimination des groupes protecteurs est réalisée après transformation du groupe ester en fonction acide.

Dans le schéma 3, il est bien entendu que l'on peut utiliser, en tant que produit (IX) de départ, un ester autre qu'un ester éthylique, par exemple un ester méthylique ou propylique ou tout autre ester d'un alkyle inférieur.

La préparation des composés de formule (IX), utiles pour la mise en oeuvre du procédé présenté dans le schéma 3, peut être réalisée selon le schéma 4, illustré à titre d'exemple pour la préparation d'un ester éthylique en tant que composé (IX).

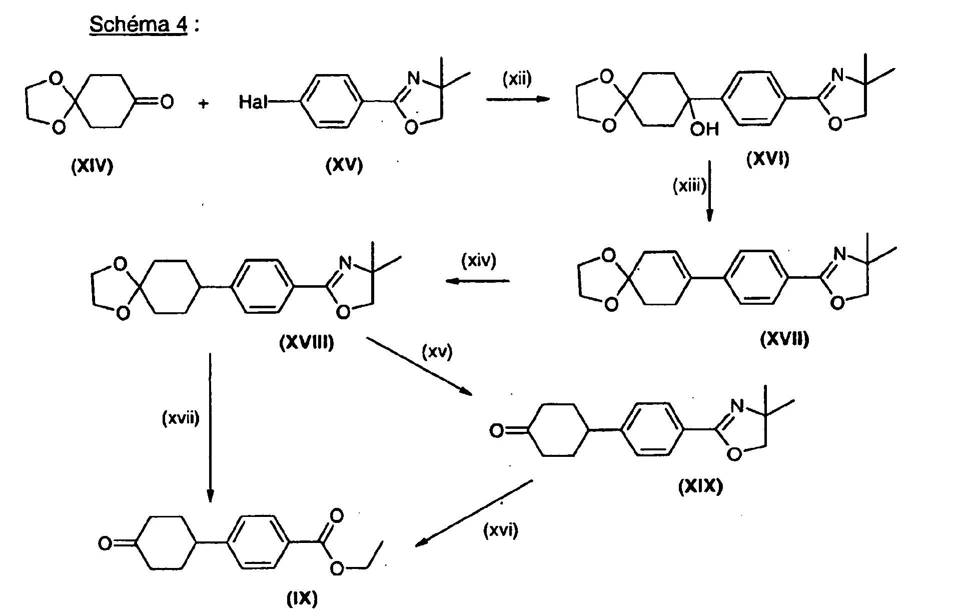

Selon le schéma 4, on procède, dans une étape (xii), à la condensation du composé de formule (XIV) avec le composé de formule (XV), dans lequel Hal représente un atome d'halogène, de préférence le brome, par exemple selon la méthode décrite par MEYERS et al. dans J. Org. Chem., 1974, 39, 2787. L'alcool intermédiaire de formule (XVI) ainsi obtenu est transformé, dans une étape (xiii), en composé (XVII) insaturé, par exemple à l'aide de SOCl₂ dans la pyridine selon la méthode décrite par GONZALES-CAMENO et al. dans Tetrahedron, 1994, 50, 10971 ou bien à l'aide de POCl₃, comme décrit par exemple dans Org. Prep. Proced. lnt., 1995, 27, 122.

Le composé (XVII) insaturé est ensuite réduit, dans une étape (xiv), en composé (XVIII) selon les méthodes conventionnelles, par exemple à l'aide d'hydrogène en présence de palladium sur charbon, dans un solvant tel que l'éthanol.

L'hydrolyse du groupe acétal du composé (XVIII) est réalisée, dans une étape (xv), de façon analogue à la réaction décrite par SZANTAY et al. dans Tetrahedron, 1996, 52(33), 11053, à savoir à l'aide d'acide chlorhydrique dans l'acétone et conduit au composé (XIX), qui est ensuite hydrolysé en composé (IX), dans une étape (xvi), selon la méthode décrite par SEEBACH et al. dans Synthesis Communications, 1982, 138 ou par NELSON et al. dans J. Org. Chem., 1994, 59(9), 2577. Alternativement, le composé (XVIII) peut être directement converti en composé (IX) par chauffage au reflux dans l'éthanol et par addition d'acide sulfurique, selon la méthode décrite par DEGRAW et al. dans J. Med. Chem., 1992, 35(2), 320 ou par TAYLOR et al. dans Heterocycles, 1996, 43(2), 323.

II est bien entendu qu'un procédé identique à celui présenté dans le schéma 4 pourrait être mis en oeuvre pour la préparation de composés (IX) sous forme d'esters autres que l'ester éthylique, en hydrolysant le composé (XVIII) à l'aide d'alcools portant des groupes alkyles différents d'un groupe éthyle.

Dans les schémas 1, 2, 3 et 4, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Préparation 1 : Trans 4-{4-[benzyl((2S)-3-{4-(benzyloxy)-3-[(méthylsulfonyl) amino]phénoxy}-2-hydroxypropyl)amino]cyclohexyl}phényl trifluorométhane sulfonate (produit intermédiaire de formule (IV) où R₁ = Pg = benzyle, R₂ = -NH-SO₂-CH₃ et R = -SO₂CF₃)

### 1.1 : Chlorhydrate du trans 4-[4-(benzylamino)cyclohexyl]phénol

Une solution de 12.2 ml de benzylamine (111 mmoles) et de 5.3 g de 4-(4'-hydroxyphényl)-cyclohexanone (27 mmoles) dans le triméthylorthoformate (100 ml) est chauffée pendant 3 h à 50°C. Les solvants sont évaporés sous pression réduite et on ajoute 100 ml d'isopropanol, puis 1.16 g de borohydrure de sodium. Le mélange réactionnel est laissé sous agitation pendant 16 h. Les solvants sont évaporés sous pression réduite et on ajoute une solution aqueuse molaire d'acide chlorhydrique jusqu'à pH=1. Le précipité est filtré et lavé à chaud par de l'acétonitrile. Le chlorhydrate du trans 4-[4-(benzylamino)cyclohexyl]phénol est obtenu sous forme d'un solide (4 g, 48%). [M+H⁺] = 282.5

### 1.2 : Trans tert-butyl benzyl[4-(4-hydroxyphényl)cyclohexyl]carbamate

Une solution de 13.7 g de trans 4-[4-(benzylamino)cyclohexyl]phénol (48.7 mmoles) et de 11.69 g de di-*tert*-butyl dicarbonate (53.5 mmoles) dans l'acétate d'éthyle (295 ml) est chauffée au reflux pendant 4 h. Les solvants sont ensuite évaporés sous pression réduite et le trans *tert*-butyl benzyl[4-(4-hydroxyphényl)cyclohexyl]carbamate est obtenu sous forme de cristaux (12.68 g, 68.2%) après purification sur gel de silice (éluant : dichlorométane/méthanol 98/2). [M+H⁺] =382.4

### 1.3 : Trans 4-{4-[benzyl(tert-butoxycarbonyl)amino]cyclohexyl}phényl trifluorométhanesulfonate

Une solution de 12.6 g de trans *tert*-butyl benzyl[4-(4-hydroxyphényl)cyclohexyl] carbamate (33 mmoles) et de 11.5 ml de 2,6-lutidine (99 mmoles) dans le dichlorométhane (125 ml) est mise sous agitation à 0°C. On ajoute ensuite 6.1 ml d'anhydride trifluorométhanesulfonique et la réaction est laissée sous agitation pendant 1 h 30. De l'eau est ajoutée et la phase aqueuse est extraite une fois au dichlorométhane. Les phases organiques sont réunies et séchées sur sulfate de sodium. Les solvants sont évaporés sous pression réduite et le trans 4-{4-[benzyl(*tert-*butoxycarbonyl)amino] cyclohexyl}phényl trifluorométhanesulfonate est obtenu sous forme de cristaux (16.43g, 97%) après purification sur gel de silice (éluant : dichlorométane). [M+H⁺] =514.4

### 1.4 : Trans 4-[4-(benzylamino)cyclohexyl]phényl trifluorométhanesulfonate

Une solution de 16.4g de trans 4-{4-[benzyl(*tert*-butoxycarbonyl)amino] cyclohexyl}phényl trifluorométhanesulfonate (32 mmoles) dans un mélange d'acide trifluoroacétique (150 ml) et de dichlorométhane (900 ml) est mise sous agitation pendant 1 h 30. Les solvants sont évaporés sous pression réduite, puis de l'eau, de l'acétate d'éthyle et une solution aqueuse d'ammoniaque sont ajoutés. La phase organique est lavée une fois à l'eau et séchée sur sulfate de sodium. Le trans 4-[4-(benzylamino)cyclohexyl]phényl trifluorométhanesulfonate est obtenu sous forme de cristaux (12.96 g, 98%) après évaporation des solvants sous pression réduite. [M+H⁺] = 414.2

### 1.5 : Trans 4-{4-[benzyl((2S)-3-{4-(benzyloxy)-3-[(tert-butoxycarbonyl) (méthylsulfonyl)amino]phénoxy}-2-hydroxypropyl)amino]cyclohexyl)phényl trifluorométhanesulfonate

Un mélange de 5.52 g de trans 4-[4-(benzylamino)cyclohexyl]phényl trifluorométhanesulfonate (13.3 mmoles) et de 5 g de 4-benzyloxy-3-(N-*tert-*butoxycarbonyl-N-méthylsulfonyl-amino)-1-((2S)-2,3-époxypropoxy)-benzène (11.2 mmoles) dans l'éthanol (72 ml) est porté au reflux pendant 5 h. Le solvant est évaporé sous pression réduite et le trans 4-(4-[benzyl((2*S*)-3-{4-(benzyloxy)-3-[(*tert-*butoxycarbonyl)(méthylsulfonyl)amino]phénoxy}-2-hydroxypropyl)amino]cyclohexyl} phényl trifluorométhanesulfonate est obtenu sous forme d'une huile (6.94 g, 72%) après purification sur gel de silice (éluant : dichlorométane/méthanol 98/2). [M+H⁺] =863.4

### 1.6 : Trans 4-{4-[benzyl((2S)-3-{4-(benzyloxy)-3-[(méthylsulfonyl)amino] phénoxy}-2-hydroxypropyl)amino]cyclohexyl}phényl trifluorométhanesulfonate

Une solution de 1.4 g de trans 4-{4-[benzyl((2*S*)-3-{4-(benzyloxy)-3-[(*tert-*butoxycarbonyl)(méthylsulfonyl)amino]phénoxy}-2-hydroxypropyl)amino]cyclohexyl} phényl trifluorométhanesulfonate (1.62 mmoles) dans un mélange d'acide trifluoroacétique (5 ml) et de dichlorométhane (45 ml) est mise sous agitation pendant 3 h 20. Les solvants sont évaporés sous pression réduite, puis de l'eau, de l'acétate d'éthyle et une solution aqueuse d'ammoniaque sont ajoutées. La phase organique est lavée une fois à l'eau et séchée sur sulfate de sodium. Le trans 4-{4-[benzyl((2S)-3-{4-(benzyloxy)-3-[(méthylsulfonyl)amino]phénoxy}-2-hydroxypropyl)amino]cyclohexyl}phényl trifluorométhanesulfonate est obtenu sous forme d'une huile (1.27 g, 98%) après évaporation des solvants sous pression réduite. [M+H⁺] =763.5

### Préparation 2: Trans (4-{4-[benzyl((2S)-3-{4-(benzyloxy)-3-[(méthylsulfonyl) amino]phénoxy}-2-hydroxypropyl)amino]cyclohexyl}phénoxy) acétate d'éthyle

### (produit intermédiaire de formule (IV) où R₁ = Pg = benzyle, R₂ = -NH-SO₂-CH₃ et R = -CH₂COOEt)

### 2.1 : Trans (4-{4-[benzyl(tert-butoxycarbonyl)amino]cyclohexyl}phénoxy) acétate d'éthyle

Une suspension de 3.82 g (10 mmoles) de trans *tert*-butyl benzyl[4-(4-hydroxyphényl)cyclohexyl]carbamate (cf. Préparation 1.2) et de 0.48 g d'hydrure de sodium à 60% (12 mmoles) dans le diméthylformamide (38 ml) est mise sous agitation pendant 25 minutes, puis on ajoute 1.44 ml de bromoacétate d'éthyle (13 mmoles). La réaction est laissée sous agitation pendant 4 h. On neutralise le milieu réactionnel par l'addition d'une solution aqueuse saturée de chlorure d'ammonium. Les solvants sont évaporés sous pression réduite et on ajoute de l'eau et du dichlorométhane. La phase organique est lavée 5 fois à l'eau, puis séchée sur sulfate de magnésium. Le trans (4-{4-[benzyl(tert-butoxycarbonyl)amino]cyclohexyl}phénoxy) acétate d'éthyle est obtenu sous forme d'un solide blanc (4.68 g, 100%) après évaporation des solvants sous pression réduite.

### 2.2 : Trans{4-[4-(benzylamino)cyclohexyl]phénoxy}acétate d'éthyle

Une solution de 4.5 g de trans (4-(4-[benzyl(*tert*-butoxycarbonyl)amino] cyclohexyl}phénoxy) acétate d'éthyle (9.6 mmoles) dans un mélange d'acide trifluoroacétique (30 ml) et de dichlorométhane (90 ml) est mise sous agitation pendant 2 h. Les solvants sont évaporés sous pression réduite, puis de l'eau, de l'acétate d'éthyle et une solution aqueuse saturée en carbonate de sodium sont ajoutés. La phase organique est lavée une fois à l'eau et séchée sur sulfate de sodium. Le trans{4-[4-(benzylamino)cyclohexyl]phénoxy}acétate d'éthyle est obtenu sous forme d'une huile (3.22 g, 88%) après évaporation des solvants sous pression réduite. [M+H⁺] =368.3

### 2.3 : Trans (4-{4-[benzyl((2S)-3-{4-(benzyloxy)-3-[(méthylsulfonyl)amino] phénoxy}-2-hydroxypropyl)amino]cyclohexyl}phénoxy) acétate d'éthyle

Un mélange de 3.22 g de trans{4-[4-(benzylamino)cyclohexyl]phénoxy)acétate d'éthyle (8.7 mmoles) et de 5 g de 4-benzyloxy-3-(N-tert-butoxycarbonyl-N-méthylsulfonyl-amino)-1-((2S)-2,3-époxypropoxy)-benzène (11.2 mmoles) dans l'éthanol (72 ml) est porté au reflux pendant 40 h. Une solution 3N d'acide chlorhydrique dans l'éthanol est ajoutée et le milieu réactionnel est chauffé à 50°C pendant 19 h. Les solvants sont évaporés sous pression réduite, puis de l'eau, du dichlorométhane et une solution aqueuse saturée en carbonate de sodium sont ajoutés. La phase aqueuse est extraite une fois au dichlorométhane. Les phases organiques sont réunies, lavées à l'eau, puis séchées sur sulfate de magnésium. Les solvants sont évaporés sous pression réduite et le trans (4-{4-[benzyl((2*S*)-3-{4-(benzyloxy)-3-[(méthylsulfonyl)amino]phénoxy}-2-hydroxypropyl)amino]cyclohexyl}phénoxy)acétate d'éthyle est obtenu sous forme d'une huile (2.7 g, 43%) après purification sur gel de silice (éluant : gradient acétate d'éthyle / heptane 30/70 à 50/50 en 40 min). [M+H⁺] =717.6

### Préparation 3 : Acide trans 4-[4-(benzyl{(2S)-3-[4-(benzyloxy)-3-[(méthylsulfonyl)amino]phénoxy]-2-hydroxypropyl}amino)cyclohexyl]benzoïque

### (Produit intermédiaire de formule (XII) où R₁ = Pg = benzyle et R₂ = -NH-SO₂-CH₃)

### 3.1: Trans 4-[4-(benzylamino)cyclohexyl]benzoate d'éthyle

Une solution de 8.51 ml de benzylamine (77.95 mmoles) et de 16 g de 4-(cyclohexanone)benzoate d'éthyle (64,96 mmoles) dans le triméthylorthoformate (192 ml) est chauffée pendant 18 h à 50°C. Les solvants sont évaporés sous pression réduite et on ajoute 267 ml d'éthanol. On ajoute ensuite 2.457 g de borohydrure de sodium. Le mélange réactionnel est laissé sous agitation pendant 2 h. Les solvants sont évaporés sous pression réduite et on ajoute du dichlorométhane et de l'eau. La phase aqueuse est extraite trois fois au dichlorométhane. Les phases organiques sont séchées sur sulfate de magnésium et concentrées sous pression réduite. Le trans 4-[4-(benzylamino)cyclohexyl]benzoate d'éthyle est obtenu sous forme d'une huile (14.69 g, 67%) après purification sur gel de silice (éluant : acétate d'éthyle/éthanol 90/10). [M+H⁺] = 282.2

### 3.2 : Trans 4-[4-(benzyl{(2S)-3-[4-(benzyloxy)-3-[(méthylsulfonyl)amino] phénoxy}-2-hydroxypropyl}amino)cyclohexyl]benzoate d'éthyle

On chauffe au reflux un mélange de 818 mg (1,82 mmoles) de 4-benzyloxy-3-(N-*tert*-butoxycarbonyl-N-méthylsulfonyl-amino)-1-((2S)-2,3-époxypropoxy)-benzène et 450 mg (1,82 mmoles) de trans 4-[4-(benzylamino)cyclohexyl]benzoate d'éthyle sous forme de base dans 15 ml d'éthanol absolu pendant 16 h. On refroidit le mélange, on y ajoute 3 ml d'une solution d'éthanol saturée en acide chlorhydrique et on chauffe à 50°C pendant 6 h. On évapore le solvant et on reprend avec un mélange de 50 ml d'une solution saturée en bicarbonate de sodium et 50 ml d'acétate d'éthyle. On lave la phase organique avec une solution aqueuse saturée en NaCl. On sèche la phase organique, on filtre et on évapore le solvant sous pression réduite. On purifie le produit brut par chromatographie sur colonne de gel de silice en éluant avec un mélange chlorure de méthylène/méthanol/NH₄OH (95/5/0,5). On obtient le composé du titre sous forme de solide blanc. [M+H⁺] = 687.

### 3.3 : Acide trans 4-[4-(benzyl{(2S)-3-[4-(benzyloxy)-3-[(méthylsulfonyl)amino] phénoxy]-2-hydroxypropyl}amino)cyclohexyl]benzoïque

On chauffe à 50°C pendant une nuit un mélange de 4.48 g (5.71 mmoles) de 4-{4-[benzyl((2S)-3-{4-(benzyloxy)-3-[(méthylsulfonyl)amino]phénoxy}-2-hydroxypropyl) amino]cyclohexyl}benzoate d'éthyle et de 38 ml d'une solution aqueuse de soude 1 N dans 114 ml d'éthanol. On évapore les solvants, on reprend dans de l'eau et on ajoute doucement une solution d'acide chlorhydrique 1 N jusqu'à pH=1. On filtre et sèche sous vide. On obtient ainsi le composé du titre sous forme d'un solide blanc (4.05 g, 94%).

Point de fusion = 160°C.

### Préparation 4 : Acide trans 4-[4-(benzyl{(2S)-3-[4-(benzyloxy)-3-(méthylsulfonyl)phénoxy]-2-hydroxypropyl)amino)cyclohexyl]benzoïque

### (Produit intermédiaire de formule (XII) où R₁ = Pg = benzyle et R₂ = -SO₂-CH₃)

### 4.1 : Trans 4-[4-(benzyl{(2S)-3-[4-(benzyloxy)-3-(méthylsulfonyl)phénoxyl-2-hydroxypropyl}amino)cyclohexyl]benzoate d'éthyle

On obtient ce produit en procédant comme décrit dans la Préparation 3.2 ci-dessus, en utilisant le trans 4-[4-(benzylamino)cyclohexyl]benzoate d'éthyle et le 4-benzyloxy-3-méthylsulfonyl-1-((2S)-2,3-époxypropoxy)-benzène, décrit dans la demande de brevet WO 99/65895, et sans y ajouter la solution d'acide chlorhydrique dans l'éthanol. [M+H⁺] = 672.

### 4.2: Acide trans 4-[4-(benzy){(2S)-3-[4-(benzyloxy)-3-(méthylsulfonyl) phénoxy]-2-hydroxypropyl}amino)cyclohexyl]benzoïque

On procède de façon similaire à la Préparation 3.3 ci-dessus, mais en utilisant le trans 4-(4-(benzyl{(2S)-3-[4-(benzyloxy)-3-(méthylsulfonyl)phénoxy]-2-hydroxypropyl} amino)cyclohexyl]benzoate d'éthyle. On obtient ainsi 8.13 g (95%) du composé du titre sous forme d'un solide blanc (point de fusion = 128-130°C).

### Exemple 1: Trans N-{5-[((2S)-3-{[4-(1,1'-biphenyl-4-yl)cyclohexyl]amino}-2-hydroxypropyl)oxy]-2-hydroxyphenyl}methanesulfonamide (composé n° 4)

### 1.1 : Trans N-[5-[((2S)-3-{benzyl[4-(1,1'-biphényl-4-yl)cyclohexyl]amino}-2-hydroxypropyl)oxy]-2-(benzyloxy)phényl]méthanesulfonamide

Un mélange de 0.3 g (0.39 mmoles) de trans 4-{4-[benzyl((2*S*)-3-{4-(benzyloxy)-3-[(méthylsulfonyl)amino]phénoxy}-2-hydroxypropyl)amino]cyclohexyl}phényl trifluorométhanesulfonate (Préparation 1), de 0.143 g d'acide phénylboronique (1.76 mmoles), de 0.091 g de tétrakis triphénylphosphine palladium (0.078 mmoles) et de 0.198 g d'hydrogénocarbonate de sodium (3.52 mmoles) dans de l'éthanol (5 ml) et de l'eau (2.5 ml) est porté au reflux pendant 1 heure. De l'eau et du dichlorométhane sont ensuite ajoutés, puis le mélange réactionnel est filtré. La phase organique est séchée sur sulfate de magnésium. Les solvants sont évaporés sous pression réduite et le trans N-[5-[((2*S*)-3-{benzyl[4-(1,1'-biphényl-4-yl)cyclohexyl]amino)-2-hydroxypropyl)oxy]-2-(benzyloxy)phényl]méthanesulfonamide est obtenu sous forme d'un solide blanc (0.16 g, 60%) après purification sur gel de silice (éluant : gradient heptane/acétate d'éthyle 95/5 à 50/50 en 20 minutes et 50/50 pendant 15 minutes). [M+H⁺] =691.5

### 1.2 : Trans N-{5-[((2S)-3-{(4-(1,1'-biphényl-4-yl)cyclohexyl]amino}-2-hydroxypropyl)oxy]-2-hydroxyphényl}méthanesulfonamide

Un mélange de 0.16g de trans N-[5-[((2S)-3-{benzyl[4-(1,1'-biphényl-4-yl)cyclohexyl]amino}-2-hydroxypropyl)oxy]-2-(benzyloxy)phényl]méthanesulfonamide (0.23 mmoles) et de 0.1 g de palladium sur charbon 10% (50% dans l'eau) dans l'éthanol (6 ml) est mis sous atmosphère d'hydrogène et agitation pendant 4 heures. Le mélange réactionnel est filtré sur célite. Les solvants sont évaporés sous pression réduite et le *N-*{5-[((2*S*)-3-{[4-(1,1'*-*biphényl-4-yl)cyclohexyl]amino}-2-hydroxypropyl)oxy]-2-hydroxy-phényl}méthanesulfonamide est obtenu sous forme d'un solide blanc (0.08 g, 68%) après purification sur gel de silice (éluant: dichlorométane/méthanol, gradient 99/1 à 85/15 en 30 minutes).

Point de fusion = 100-110°C ; [M+H⁺]= 511.4 ; RMN ¹H (DMSO-D6 + D₂O, 200 MHz) : 1.05-1.35 (m, 2H), 1.4-1.6 (m, 2H), 1.7-2 (m, 5H), 2.4-2.8 (m, 3H), 2.85 (s, 3H), 3.7-3.85 (m, 3H), 6.52 (dd,1H), 6.7-6.82 (m, 2H), 7.2-7.65 (m, 9H).

### Exemple 2: Trans N-{2-hydroxy-5-[((2S)-2-hydroxy-3-{[4-(4-hydroxyphényl) cyclohexyl]amino}propyl)oxy]phényl}méthanesulfonamide (composé n° 1)

### 2.1 : Trans N-[5-[((2S)-3-{benzyl[4-(4-hydroxyphényl)cyclohexyl]amino}-2-hydroxypropyl)oxy]-2-(benzyloxy)phényl]méthanesulfonamide

On chauffe au reflux un mélange de 1.52 g (3,38 mmoles) de 4-benzyloxy-3-(N-tert-butoxycarbonyl-N-méthylsulfonyl-amino)-1-((2S)-2,3-époxypropoxy)-benzène et 1 g (3.55 mmoles) de trans 4-[4-(benzylamino)cyclohexyl]phénol sous forme de base dans 20 ml d'éthanol absolu pendant 16 h. On refroidit le mélange, on y ajoute 20 ml d'une solution d'éthanol saturée en acide chlorhydrique et on chauffe à 50°C pendant 1 h. On évapore le solvant et on reprend avec un mélange de 100 ml d'une solution saturée en bicarbonate de sodium et 100 ml d'acétate d'éthyle. On lave la phase organique avec une solution aqueuse saturée en NaCl. On sèche la phase organique, on filtre et on évapore le solvant sous pression réduite. On purifie le produit brut par chromatographie sur colonne de gel de silice en éluant avec un mélange heptane/acétate d'éthyle (55/45). On obtient le composé du titre sous forme de solide blanc (1.8 g, 84%). [M+H]⁺ = 631.5.

### 2.2 : Trans N-{2-hydroxy-5-[((2S)-2-hydroxy-3-{[4-(4-hydroxyphényl) cyclohexyl]amino}propyl)oxy]phényl}méthanesulfonamide

Un mélange de 0.8 g (1.26 mmoles) de trans *N*-[5-[((2S)-3-{benzyl[4-(4-hydroxyphényl)cyclohexyl]amino}-2-hydroxypropyl)oxy]-2-(benzyloxy)phényl]méthane-sulfonamide et de 0.2 g de palladium sur charbon 10% (50% dans l'eau) dans l'éthanol (40 ml) est mis sous atmosphère d'hydrogène et agitation pendant 2 h. Le mélange réactionnel est filtré sur célite. Les solvants sont évaporés sous pression réduite et le trans *N*-{2-hydroxy-5-[((2*S*)-2-hydroxy-3-{[4-(4-hydroxyphényl)cyclohexyl}amino}propyl) oxy]phényl}méthanesulfonamide est obtenu sous forme d'un solide blanc (0.24 g, 67%) après purification sur gel de silice (éluant : dichlorométane/méthanol, gradient 95/5 à 80/20 en 15 min).

Point de fusion = 85-90°C ; [M+H⁺] = 451.4 ; RMN ¹H (DMSO-D6, 300 MHz) :1.15 (dd, 2H), 1.38 (dd, 2H), 1.68-1.8 (m, 2H), 1.85-2 (m, 2H), 2.25-2.52 (m, 3H), 2.61 . (dd,1 H), 2.75 (dd,1 H), 2.91 (s, 3H), 3.7-3.9 (m, 3H), 6.55 (dd, 1 H), 6.65 (d, 2H), 6.75-6.8 (m, 2H), 6.95 (d, 2H).

### Exemple 3 : Trans (4-{4-[((2S)-2-hydroxy-3-{4-hydroxy-3-[(méthylsulfonyl) amino]phénoxy}propyl)amino]cyclohexyl}phénoxy)acétate d'éthyle (composé n° 2)

Un mélange de 0.2 g de trans (4-{4-[benzyl((2*S*)-3-{4-(benzyloxy)-3-[(méthylsulfonyl)amino]phénoxy}-2-hydroxypropyl)amino]cyclohexyl}phenoxy) acétate d'éthyle (0.26 mmoles) et de 0.25 g de palladium sur charbon 10% (50% dans l'eau) dans l'éthanol (16 ml) est mis sous atmosphère d'hydrogène et agitation pendant 7 h. Le mélange réactionnel est filtré sur célite. Les solvants sont évaporés sous pression réduite et le trans (4-{4--[((2*S*)-2-hydroxy-3-{4-hydroxy-3-[(méthylsulfonyl)amino] phénoxy}propyl) amino]cyclohexyl}phénoxy)acétate d'éthyle est obtenu sous forme d'un solide blanc (0.08 g, 54%) après purification sur gel de silice (éluant : dichlorométane / méthanol, gradient 99/1 à 85/15 en 30 min).

Point de fusion = 60-70°C ; [M+H⁺]= 537.6 ; RMN ¹H (DMSO-D6 + D₂O, 200 MHz) : 1.1- 1.5 (m, 4H), 1.2 (t, 3H),1.65-1.8 (m, 2H), 1.85-2 (m, 2H), 2.3-2.85 (m, 4H), 2.93 (s, 3H), 3.7-3.9 (m, 3H), 4.18 (q, 2H), 4.7 (s, 2H), 6.55 (dd,1H), 6.7-6.85 (m, 4H), 7.08 (d, 2H).

### Exemple 4 : Acide trans (4-{4-[((2S)-2-hydroxy-3-{4-hydroxy-3-[(méthylsulfonyl)amino]phénoxy}propyl)amino]cyclohexyl}phénoxy)acétique (composé n° 3)

### 4.1 : Acide trans (4-{4-[benzyl((2S)-3-{4-(benzyloxy)-3-[(méthylsulfonyl) amino]phénoxy}-2-hydroxypropyl)amino]cyclohexyl}phénoxy)acétique

Un mélange de 0.36 g de trans (4-{4-[benzyl((2*S*)-3-{4-(benzyloxy)-3-[(méthylsulfonyl)amino]phénoxy}-2-hydroxypropyl)amino]cyclohexyl}phénoxy)acétate d'éthyle (0.49 mmoles) et de 6 ml d'une solution aqueuse molaire de soude dans l'éthanol (10 ml) est mis à chauffer à 45°C pendant 22 h. Les solvants sont ensuite évaporés sous pression réduite et de l'eau est ajoutée. On ajoute ensuite, jusqu'à pH = 1, une solution aqueuse molaire d'acide chlorhydrique. Le produit du titre est obtenu sous forme d'un solide blanc (0.3 g, 89%) après filtration et séchage sous vide. [M+H⁺]= 689.7

### 4.2: Acide trans (4-{4-[((2S)-2-hydroxy-3-{4-hydroxy-3-[(méthylsulfonyl) amino]phénoxy}propyl)amino]cyclohexyl}phénoxy)acétique

Un mélange de 0.3 g d'acide trans (4-{4-[benzyl((2*S*)-3-{4-(benzyloxy)-3-[(méthylsulfonyl)amino]phénoxy}-2-hydroxypropyl)amino]cyclohexyl}phénoxy)acétique (0.44 mmoles) et de 0.20 g de palladium sur charbon 10% (50% dans l'eau) dans un mélange de tétrahydrofurane (5 ml) et d'éthanol (5ml) est mis sous atmosphère d'hydrogène et sous agitation pendant 4 h. Le mélange réactionnel est filtré sur célite. Les solvants sont évaporés sous pression réduite et l'acide (4-{4-[((2S)-2-hydroxy-3-{4-hydroxy-3-[(méthylsulfonyl)amino]phénoxy}propyl)amino]cyclohexyl}phénoxy)acétique est obtenu sous forme d'un solide blanc (0.07 g, 32%) après purification sur silice greffée C18 (éluant : eau/acétonitrile, gradient 95/5 à 5/95 en 15 min).

Point de fusion = 160-170°C ; [M+H⁺]= 509.6; RMN ¹H (DMSO-D6 + D₂O, 200 MHz) : 1.1-1.45 (m, 4H), 1.6-2 (m, 4H), 2.1-2.25 (m, 1H), 2.6-3.1 (m, 4H), 2.95 (s, 3H), 3.75-3.9 (m, 2H), 4-4.15 (m, 1 H), 4.28(s, 2H), 6.55-6.82 (m, 5H), 7.05 (d, 2H).

### Exemple 5 : Trans N-(4-{4-[((2S)-2-hydroxy-3-{4-hydroxy-3-[(méthylsulfonyl) amino]phénoxy}propyl)amino]cyclohexyl}benzoyl)-L-valinate d'éthyle

### (composé n° 13)

### 5.1 : Trans (2S)-2-[(4-{4-[benzyl((2S)-3-{4-(benzyloxy)-3-[(méthylsulfonyl) amino]phénoxy}-2-hydroxypropyl)amino]cyclohexyl}benzoyl)amino]-3-méthylbutanoate d'éthyle

Une solution de 0.3 g (0.45 mmol) d'acide trans 4-{4-[benzyl((2S)-3-{4-(benzyloxy)-3-[(méthylsulfonyl)amino]phénoxy}-2-hydroxypropyl)amino]cyclohexyl} benzoïque (Préparation 3), de 0.78 g (0.91 mmol) de 1-hydroxy-benzotriazole, de 0.175 g (0.91 mmol) de chlorhydrate de 1-éthyl-3-(3-diméthylamino-propyl) carbodiimide, de 0.19 ml de triéthylamine et de 0.165 g (0.91 mmol) de chlorhydrate de l'ester éthylique de la L-valine dans 5 ml de dichlorométhane est mise sous agitation pendant 24 h. Les solvants sont évaporés sous pression réduite. Du dichlorométhane et de l'eau sont ajoutés et la phase organique est lavée trois fois à l'eau. Les phases organiques sont séchées sur sulfate de magnésium et concentrées sous pression réduite. Le trans (2S)-2-[(4-{4-[benzyl((2*S*)-3-{4-(benzyloxy)-3-[(méthylsulfonyl)amino]phénoxy}-2-hydroxypropyl) amino]cyclohexyl}benzoyl)amino]-3-méthylbutanoate d'éthyle est obtenu sous forme d'une huile jaune (0.35 g, 98%). [M+H⁺] = 786

### 5.2 : Trans N-(4-{4-[((2S)-2-hydroxy-3-{4-hydroxy-3-[(méthylsulfonyl)amino] phénoxy}propyl)amino]cyclohexyl}benzoyl)-L-valinate d'éthyle

Une suspension de 1.35 g (0.35 mmol) de trans (2S)-2-[(4-{4-[benzyl((2*S*)-3-{4-(benzyloxy)-3-[(méthylsulfonyl)amino]phénoxy}-2-hydroxypropyl)amino]cyclohexyl} benzoyl)amino]-3-méthylbutanoate d'éthyle et de 0.177 g de palladium sur charbon (10% Pd, 50% dans l'eau) dans 15 ml d'éthanol est mise sous atmosphère d'hydrogène et sous agitation pendant 3 h. Le catalyseur est ensuite filtré et les solvants évaporés sous pression réduite. Le composé du titre est obtenu sous forme d'un solide blanc (0.138 g, 44%) après purification sur gel de silice (éluant : gradient dichlorométhane / méthanol / ammoniaque, 99/1/0.1 à 85/15/1.5 en 30 min).

Point de fusion = 65-75°C ; RMN ¹H (DMSO-D6 + D₂O, 200 MHz) : 0.92(t, 3H), 1.1-1.35 (m, 8H), 1.35-1.6 (m, 2H), 1.7-2.25 (m, 5H), 2.4-2.8 (m, 4H), 2.92 (s, 3H), 3.6-3.9 (m, 3H), 4-4.2 (m, 2H), 4.25(t, 1 H), 6.6 (dd, 1 H), 6.68-6.8 (m, 2H), 7.32 (d, 2H), 7.78 (d, 2H).

### Exemple 6 : Trans N-(4-{4-[((2S)-2-hydroxy-3-{4-hydroxy-3-[(méthylsulfonyl), amino]phénoxy}propyl)amino]cyclohexyl}benzoyl)-L-phénylalanine (composé n°19)

### 6.1: Trans N-(4-{4-[benzyl((2S)-3-{4-(benzyloxy)-3-[(méthylsulfonyl)amino] phénoxy}-2-hydroxypropyl)amino]cyclohexyl}benzoyl)-L-phénylalaninate d'éthyle

Une solution de 0.3g (0.45 mmol) d'acide trans 4-{4-[benzyl((2S)-3-{4-(benzyloxy)-3-[(méthylsulfonyl)amino]phénoxy}-2-hydroxypropyl)amino]cyclohexyl} benzoïque (Préparation 3), de 0.78 g (0.91 mmol) de 1-hydroxy-benzotriazole, de 0.175 g (0.91 mmol) de chlorhydrate de 1-éthyl-3-(3-diméthylamino-propyl)carbodiimide, de 0.19 ml de triéthylamine et de 0.165 g (0.91 mmol) de chlorhydrate de l'ester éthylique de la L-phénylalanine dans 5 ml de dichlorométhane est mise sous agitation pendant 24 h. Les solvants sont évaporés sous pression réduite. Du dichlorométhane et de l'eau sont ajoutés et la phase organique est lavée trois fois à l'eau. Les phases organiques sont séchées sur sulfate de magnésium et concentrées sous pression réduite. Le produit est obtenu sous forme d'un solide blanc (0.350 g, 92%) après purification sur gel de silice (éluant : gradient dichlorométhane/méthanol 100/00 à 90/10 en 35 min). [M+H⁺] = 835

### 6.2: Trans N-(4-{4-[benzyl((2S)-3-(4-(benzyloxy)-3-[(méthylsulfonyl)amino] phénoxy}-2-hydroxypropyl)amino]cyclohexyl}benzoyl)-L-phénylalanine

Un mélange de 0.35g de trans *N*-(4-{4-[benzyl((2*S*)-3-(4-(benzyloxy)-3-[(méthylsulfonyl)amino]phénoxy}-2-hydroxypropyl)amino]cyclohexyl}benzoyl)-L-phénylalaninate d'éthyle (0.42 mmoles) et de 1.7 ml d'une solution aqueuse molaire de soude dans l'éthanol (9.3 ml) est mis à chauffer à 45°C pendant 3 h. Les solvants sont ensuite évaporés sous pression réduite et de l'eau est ajoutée. On ajoute ensuite jusqu'à pH = 1 une solution aqueuse molaire d'acide chlorhydrique. Le produit du titre est obtenu sous forme d'un solide blanc (0.2 g, 59%) après filtration et séchage sous vide. [M+H⁺] = 807

### 6.3 : Trans N-(4-{4-[((2S)-2-hydroxy-3-{4-hydroxy-3-[(méthylsulfonyl)amino] phénoxy}propyl)amino]cyclohexyl}benzoyl)-L-phénylalanine

Un mélange de 0.2 g de trans *N*-(4-{4-[benzyl((2*S*)-3-{4-(benzyloxy)-3-[(méthylsulfonyl)amino]phénoxy}-2-hydroxypropyl)amino]cyclohexy})benzoyl)-L-phénylalanine (0.248 mmoles) et de 0.1 g de palladium sur charbon 10% (50% dans l'eau) dans l'éthanol (15 ml) est mis sous atmosphère d'hydrogène et agitation pendant 3 h. Le mélange réactionnel est filtré sur célite. Les solvants sont évaporés sous pression réduite et le trans *N*-(4-{4-[((2*S*)-2-hydroxy-3-{4-hydroxy-3-[(méthylsulfonyl) amino]phénoxy}propyl)amino]cyclohexyl}benzoyl)-L-phénylalanine est obtenu sous forme d'un solide blanc (0.049 g, 31%) après purification sur silice greffée C18 (éluant : eau/acétonitrile, gradient 95/5 à 5/95 en 15 min).

Point de fusion >230°C ; [M+H⁺]= 626.4 ; RMN ¹H (DMSO-D6 + D₂O, 200 MHz) : 1.1-1.65 (m, 6H), 1.9-2.1 (m, 2H), 2.2.2.38 (m, 1H), 2.7-3.3 (m, 4H), 2.92 (s, 3H), 3.75-3.9 (m, 3H), 4-4.2 (m, 1H), 4.25-4.4(m, 1 H), 6.65 (dd, 1H), 6.75-6.9 (m, 2H), 7-7.3 (m, 7H), 7.68 (d, 2H).

### Exemple 7 : Trans 4-[4-({(2S)-2-hydroxy-3-[4-hydroxy-3-(méthylsulfonyl) phénoxy]propyl}amino)cyclohexyl]-N-phénylbenzohydrazide (composé n° 26)

### 7.1 : Trans 4-[4-(benzyl{(2S)-3-[4-(benzyloxy)-3-(méthylsulfonyl)phénoxy]-2-hydroxypropyl}amino)cyclohexyl]-N-phénylbenzohydrazide

Une solution de 0.3 g (0.441 mmol) d'acide trans 4-[4-(benzyl{(2S)-3-[4-(benzyloxy)-3-(méthylsulfonyl)phénoxy]-2-hydroxypropyl)amino)cyclohexyl]benzoïque (Préparation 4), de 0.78 g (0.91 mmol) de 1-hydroxy-benzotriazole, de 0.169 g (0.88 mmol) de chlorhydrate de 1-éthyl-3-(3-diméthylamino-propyl)carbodiimide, de 0.184 ml de triéthylamine et de 0.095 g (0.88 mmol) de phénylhydrazine dans un mélange de 4 ml de dichlorométhane et de 0.8 ml d'acétonitrile est mise sous agitation pendant 48 heures. Les solvants sont évaporés sous pression réduite. Du dichlorométhane et de l'eau sont ajoutés et la phase organique est lavée trois fois à l'eau. Les phases organiques sont séchées sur sulfate de magnésium et concentrées sous pression réduite. Le produit est obtenu sous forme d'un solide blanc (0.166 g, 50%) après purification sur gel de silice (éluant : gradient heptane/acétate d'éthyle 60/40 pendant 10 min, 60/40 à 40/60 en 10 min, puis 40/60 pendant 20 min). [M+H⁺] = 734.7

### 7.2: Trans 4-[4-({(2S)-2-hydroxy-3-[4-hydroxy-3-(méthylsulfonyl)phénoxy] propyl}amino)cyclohexyl]-N-phénylbenzohydrazide

Un mélange de 0.166 g de trans 4-[4-(benzyl{(2*S*)-3-[4-(benzyloxy)-3-(méthylsulfonyl)phénoxy]-2-hydroxypropyl}amino)cyclohexyl]-*N*-phénylbenzohydrazide (0.226 mmoles) et de 0.1 g de palladium sur charbon 10% (50% dans l'eau) dans un mélange d'éthanol (3.9 ml) et de tétrahydrofurane (2.93 ml) est mis sous atmosphère d'hydrogène et sous agitation pendant 48 h. Le catalyseur est ensuite filtré et les solvants évaporés sous pression réduite. Le composé du titre est obtenu sous forme d'un solide blanc (0.02 g, 16%) après purification sur gel de silice (éluant : gradient dichlorométhane / méthanol / ammoniaque, 99/1/0.1 à 85/15/1.5 en 30 min).

Point de fusion = 125°C ; [M+H⁺]= 554 ; RMN ¹H (DMSO-D6, 200 MHz) : 1.0-1.6 (m, 4H), 1.7-2.1 (m, 4H), 2.35-2. 8 (m, 4H), 2.7-3.3 (m, 4H), 3.2 (s, 3H), 3.7-3.95 (m, 3H), 4-4.2 (m, 1 H), 4.25-4.4(m, 1 H), 6.65 (dd, 1 H), 6.6-7.4 (m, 9H), 7.75-7.9 (m, 3H).

### Exemple 8: Trans N-(4-fluorobenzyl)-4-[4-({(2S)-2-hydroxy-3-[4-hydroxy-3-(méthylsulfonyl)phénoxy]propyl}amino)cyclohexyl]benzamide (composé n° 25)

### 8.1 : Trans 4-[4-(benzyl{(2S)-3-[4-(benzyloxy)-3-(méthylsulfonyl)phénoxy]-2-hydroxypropyl}amino)cyclohexyl]-N-(4-fluorobenzyl)benzamide

Une solution de 0.3 g (0.441 mmol) d'acide trans 4-[4-(benzyl{(2S)-3-[4-(benzyloxy)-3-(méthylsulfonyl)phénoxy]-2-hydroxypropyl}amino)cyclohexyl]benzoïque (Préparation 4), de 0.78 g (0.91 mmol) de 1-hydroxy-benzotriazole, de 0.169 g (0.88 mmol) de chlorhydrate de 1-éthyl-3-(3-diméthylamino-propyl)carbodiimide, de 0.184 ml de triéthylamine et de 0.11 g (0.88 mmol) de 4-fluorobenzylamine dans un mélange de 4 ml de dichlorométhane et de 0.8 ml d'acétonitrile est mise sous agitation pendant 48 h. Les solvants sont évaporés sous pression réduite. Du dichlorométhane et de l'eau sont ajoutés et la phase organique est lavée trois fois à l'eau. Les phases organiques sont séchées sur sulfate de magnésium et concentrées sous pression réduite. Le produit est obtenu sous forme d'un solide blanc (0.232 g, 70%) après purification sur gel de silice (éluant : gradient heptane/acétate d'éthyle 60/40 pendant 10 min, 60/40 à 40/60 en 10 min, puis 40/60 pendant 20 min). [M+H⁺] = 751.5

### 8.2 : Trans N-(4-fluorobenzyl)-4-[4-({(2S)-2-hydroxy-3-[4-hydroxy-3-(méthylsulfonyl)phénoxy]propyl}amino)cyclohexyl]benzamide

Un mélange de 0.166g de trans 4-[4-(benzyl{(2S)-3-[4-(benzyloxy)-3-(méthylsulfonyl)phénoxy]-2-hydroxypropyl}amino)cyclohexyl]-N-(4-fluorobenzyl) benzamide (0.226 mmoles) et de 0.117 g de palladium sur charbon 10% (50% dans l'eau) dans un mélange d'éthanol (5.3 ml) et de tétrahydrofurane (4.02 ml) est mis sous atmosphère d'hydrogène et agitation pendant 48 heures. Le catalyseur est ensuite filtré et les solvants évaporés sous pression réduite. Le composé du titre est obtenu sous forme d'un solide blanc (0.92 g, 52%) après purification sur gel de silice (éluant : gradient, dichlorométhane / méthanol / ammoniaque, 99/1/0.1 à 85/15/1.5 en 30 min).

Point de fusion = 110°C ; [M+H⁺]= 571 ; RMN ¹H (DMSO-D6, 500 MHz) : 1.12-1.2 (m, 2H), 1.4-1.5(m, 2H), 1.7-1.8 (m, 2H), 1.9-2 (m, 2H), 2.45-2.55 (m, 2H), 2.6-2.68 (m, 1 H), 2.7-2.8(m, 1 H), 3.22 (s, 3H), 3.75-3.95 (m, 3H), 4.4 (s, 2H), 6.88 (dd, 1 H), 7.08-7.2 (m, 4H), 7.28-7.3 (m, 3H), 7.74 (d, 2H).

### Exemple 9 : Trans 4-{4-[((2S)-2-hydroxy-3-{4-hydroxy-3-[(méthylsulfonyl) amino]phénoxy}propyl)amino]cyclohexyl}-N-[2-(1H-indol-3-yl)éthyl]benzamide

### (composé n° 29)

### 9.1 :Trans 4-{4-[benzyl((2S)-3-{4-(benzyloxy)-3-[(méthylsulfonyl)amino] phénoxy}-2-hydroxypropyl)amino]cyclohexyl}-N-[2-(1H-indol-3-yl)éthyl]benzamide

Une solution de 0.3 g (0.45 mmol) d'acide trans 4-{4-[benzyl((2S)-3-{4-(benzyloxy)-3-[(méthylsulfonyl)amino]phénoxy}-2-hydroxypropyl)amino]cyclohexyl} benzoïque (Préparation 3), de 0.78g (0.91 mmol) de 1-hydroxy-benzotriazole, de 0.175g (0.91 mmol) de chlorhydrate de 1-éthyl-3-(3-diméthylamino-propyl)carbodiimide, de 0.19 ml de triéthylamine et de 0.138 g (0.86 mmol) de tryptamine dans un mélange de 4 ml de dichlorométhane et 0.8 ml d'acétonitrile est mise sous agitation pendant 24 h. Les solvants sont évaporés sous pression réduite. Du dichlorométhane et de l'eau sont ajoutés et la phase organique est lavée trois fois à l'eau. Les phases organiques sont séchées sur sulfate de magnésium et concentrées sous pression réduite. Le composé du titre est obtenu sous forme d'un solide blanc (0.246 g, 58%) après purification sur gel de silice (éluant : gradient heptane/acétate d'éthyle 60/40 pendant 10 min, 60/40 à 40/60 en 10 min, puis 40/60 pendant 20 min). [M+H⁺] = 801.6

### 9.2 : Trans 4-{4-[((2S)-2-hydroxy-3-{4-hydroxy-3-[(méthylsulfonyl)amino] phénoxy)propyl)amino]cyclohexyl)-N-[2-(1H-indol-3-yl)éthyl]benzamide

Une suspension de 0.246 g (0.3 mmol) de trans 4-{4-[benzyl((2*S*)-3-{4-(benzyloxy)-3-[{méthylsulfonyl)amino]phénoxy}-2-hydroxypropyl)amino]cyclohexyl}-*N-*[2-(1H-indol-3-yl)éthyl]benzamide et de 0.332 g de palladium sur charbon (10% Pd, 50% dans l'eau) dans un mélange de 5.4 ml d'éthanol et 4 ml de tétrahydrofurane est mise sous atmosphère d'hydrogène et sous agitation pendant 3 h. Le catalyseur est ensuite filtré et les solvants évaporés sous pression réduite. Le composé du titre est obtenu sous forme d'un solide blanc (0.076 g, 40%) après purification sur gel de silice (éluant : gradient dichlorométhane / méthanol / ammoniaque, 99/1/0.1 à 85/15/1.5 en 30 min).

Point de fusion = 125°C ; [M+H⁺] = 621 ; RMN ¹H (DMSO-D6, 200 MHz) : 1-1.2 (m, 2H), 1.3-1.55(m, 2H), 1.7-2 (m, 4H), 2.4-3 (m, 6H), 2.9 (s, 3H), 3.4-3.6 (m, 2H), 3.7-3.8(m, 1 H), 3.8-3.95 (m, 3H), 6.6 (dd, 1 H), 6.7-6.85 (m, 2H), 6.9-7.2 (m, 3H), 7.25-7.4 (m, 3H), 7.55 (d, 1 H), 7.75 (d, 2H), 8.4-8.5 (m, 1H).

### Exemple 10: Trans 2-{4-[4-({(2S)-3-[3-(butylsulfinyl)-4-hydroxyphénoxy]-2-hydroxypropyl}amino)cyclohexyl]phénoxy}-2,2-diméthylacétate d'éthyle (composé n°34)

### 10.1: Hydrochlorure de trans 2-[4-(4-aminocyclohexyl)phénoxy]-2,2-diméthylacétate d'éthyle

Un mélange de 1.9 g de trans 2-{4-[4-(benzylamino)cyclohexyl]phénoxy}-2,2-diméthylacétate d'éthyle sous forme de base (4.8 mmol) et de 0.1 g de palladium sur charbon 10 % dans de l'éthanol (75 ml) est mis sous atmosphère d'hydrogène à 40°C et agitation pendant 7h. Le mélange réactionnel est filtré sur célite. Le solvant est évaporé sous pression réduite et le trans 2-[4-(4-aminocyclohexyl)phenoxy]-2,2-dimethylacétate d'éthyle est obtenu (0.660 g) sous forme d'huile jaune qui est dissoute dans de l'éthanol (5 ml). On acidifie avec une solution de HCl (9N) dans l'éthanol et on filtre le solide ainsi obtenu (0.530 g, 72%). Point de fusion : 240-242.

### 10.2 : Trans 2-{4-[4-({(2S)-3-[4-(benzyloxy)-3-(butylsulfinyl)phénoxy]-2-hydroxypropyl}amino)cyctohexyl]phénoxy}-2,2-diméthylacétate d'éthyle

Un mélange de 0.742 g de (2S)-2-{[4-(benzyloxy)-3-(butylsulfinyl)phénoxylméthyl)oxirane (obtenu par analogie avec la méthode décrite dans WO 99 65895 mais en partant du butylsulfoxyde au lieu du méthylsulfoxyde et en utilisant le (S)(+)glycidylnosilate ; [α]_{D} = +1.9 (C = 1%, MeOH)) (2.06 mmol) et de 0.666 g de 2-[4-(4-aminocyclohexyl)phénoxy]-2,2-diméthylacétate d'éthyle (2.18 mmol) dans de l'éthanol (25 ml) est chauffé à reflux pendant une nuit. On évapore le solvant sous pression réduite et on purifie le produit obtenu par flash chromatographie en éluant avec un mélange de chlorure de méthylène / méthanol / ammoniaque (95:5:0.5). On obtient le produit du titre sous forme d'huile jaune (0.730 g, 53%)

### 10.3 : Trans 2-{4-[4-({(2S)-3-[3-(butylsulfinyl)-4-hydroxyphénoxy]-2-hydroxypropyl}amino)cyclohexyl]phénoxy}-2,2-diméthylacétate d'éthyle

Une solution de 0.720 g de trans 2-{4-[4-({(2S)-3-[4-(benzyloxy)-3-(butylsulfinyl)phénoxy]-2-hydroxypropyl}amino)cyclohexyl]phénoxy}-2,2-diméthylacétate d'éthyle (1.08 mmol) dans de l'acide trifloroacétique (12 ml) est chauffé à 60°C pendant 3 heures. On évapore le solvant et on traite avec une solution de bicarbonate de sodium et de l'acétate d'éthyle. La phase organique est séparée, séchée, filtrée et évaporée sous vide. On purifie le produit ainsi obtenu par flash chromatographie en éluant avec un mélange de chlorure de méthylène / méthanol / ammoniaque (9:1:0.1). On obtient le produit du titre sous forme d'un solide (0.31 g, 50%).

Point de fusion = 58-60°C; [M+H⁺] = 576; RMN ¹H (DMSO-D6 313K) : 0.86 (3H; t; J 7Hz); 1.17 (3H; t; J 7Hz); 1.05-1.24 (2H; m); 1.27-1.46 (2H; m); 1.49 (6H; s); 1.60-1.72 (1H;m); 1.77 (2H; bd; J 13Hz); 1.96 (2H; bd; J 13Hz); 2.30-2.47 (2H; m); 2.58-2.83 (3H; m); 2.93-3.09 (1H; m); 3.74-3.89 (2H; m); 3.89-3.98 (1H; m); 4.16 (2H; q; J 7Hz); 6.71 (2H; m); 6.81 (1H; d; J 9 Hz); 6.92 (1H; dd; Ja 9 Hz; Jb 3Hz); 7.07 (1 H; d; J 3 Hz); 7.10 (2H; m).

Le tableau qui suit illustre les structures chimiques et les propriétés physiques de quelques composés selon l'invention. Dans ce tableau :
- dans la colonne « sel », « - » représente un composé sous forme de base libre,
- Me, Et, iPr, nBu, iBu, Ph et Bn représentent respectivement les groupes méthyle, éthyle, isopropyle, butyle, isobutyle, phényle et benzyle.

**Tableau**

| 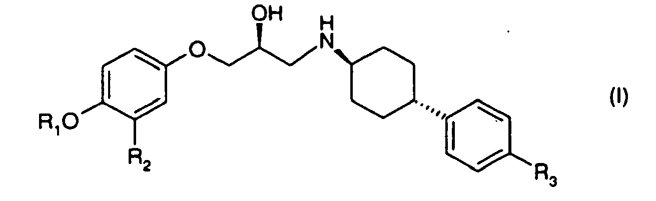 | | | | | |
|---|---|---|---|---|---|
| N° | R₁ | R₂ | R₃ | Sel | Point de fusion (°C) |
| 1 | H | -NHSO₂CH₃ | -OH | - | 85-90 |
| 2 | H | -NHSO₂CH₃ | 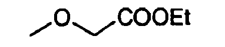 | - | 60-70 |
| 3 | H | -NHSO₂CH₃ | 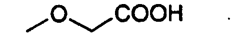 | - | 160-170 |
| 4 | H | -NHSO₂CH₃ | 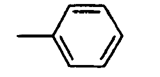 | - | 100-110 |
| 5 | H | -NHSO₂CH₃ | 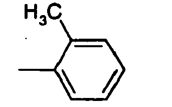 | - | 90-95 |
| 6 | H | -NHSO₂CH₃ | 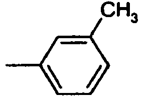 | - | 95-100 |
| 7 | H | -NHSO₂CH₃ | 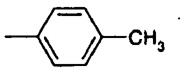 | - | 140-150 |
| 8 | H | -NHSO₂CH₃ | 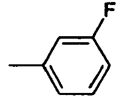 | - | 105-110 |
| 9 | H | -NHSO₂CH₃ | 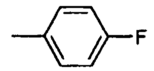 | - | 120-125 |
| 10 | H | -NHSO₂CH₃ | 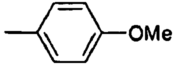 | - | 165-175 |
| 11 | H | -NHSO₂CH₃ | 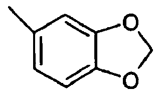 | - | 215-220 |
| 12 | H | -NHSO₂CH₃ | 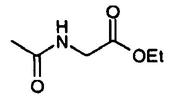 | - | 64-69 |
| 13 | H | -NHSO₂CH₃ | 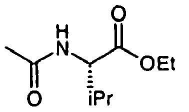 | - | 65-75 |
| 14 | H | -NHSO₂CH₃ | 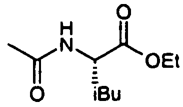 | - | 65-80 |
| 15 | H | -NHSO₂CH₃ | 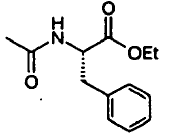 | - | 73-83 |
| 16 | H | -NHSO₂CH₃ | 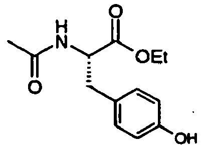 | - | 70-80 |
| 17 | H | -NHSO₂CH₃ | 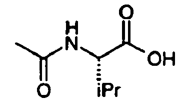 | - | 150-180 |
| 18 | H | -NHSO₂CH₃ | 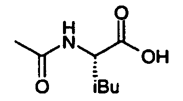 | - | > 250 |
| 19 | H | -NHSO₂CH₃ | 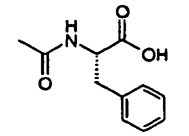 | - | > 230 |
| 20 | H | -NHSO₂CH₃ | 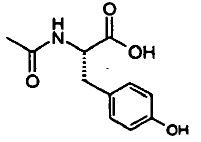 | - | 170-210 |
| 21 | H | -NHSO₂CH₃ | 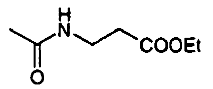 | pamoate | 110-150 |
| 22 | H | -NHSO₂CH₃ | 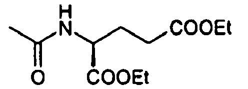 | - | 55-65 |
| 23 | H | -NHSO₂CH₃ | 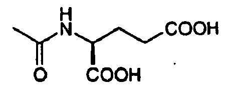 | - | 160-200 |
| 24 | H | -NHSO₂CH₃ | 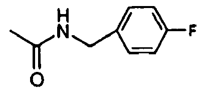 | - | 75-85 |
| 25 | H | -SO₂CH₃ | 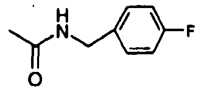 | - | 110 |
| 26 | H | -SO₂CH₃ | 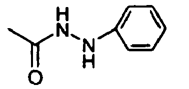 | - | 125 |
| 27 | H | -SO₂CH₃ | 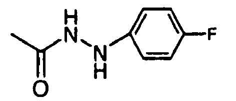 | - | 125 |
| 28 | H | -SO₂CH₃ | 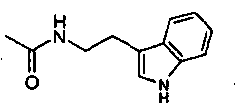 | - | 115 |
| 29 | H | NHSO₂CH₃ | 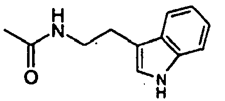 | - | 125 |
| 30 | H | -SO₂CH₃ | 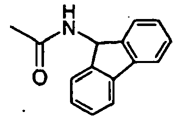 | - | 141 |
| 31 | H | -SO₂CH₃ | 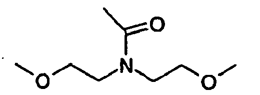 | - | 80 |
| 32 | H | NHSO₂CH₃ | 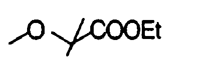 | - | 45-48 |
| 33 | H | -NHSO₂Ph | 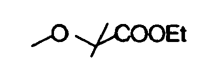 | - | 72-74 |
| 34 | H | -SO(nBu) | 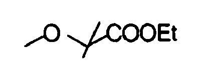 | - | 58-60 |
| 35 | H | -SO(nBu) | 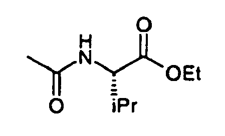 | - | 76-78 |

Les composés selon l'invention ont fait l'objet d'essais pharmacologiques permettant de déterminer leur effet activité agoniste vis-à-vis des récepteurs bêta-3.

L'activité agoniste vis-à-vis des récepteurs bêta-3 (traduite par la production de cAMP induite par le composé testé) a été étudiée à l'aide de préparations membranaires de cellules SKNMC (cellules de neuroblastomes humains) en présence d'antagonistes sélectifs bêta-1 et bêta-2 (CGP20712 et ICI118551, tous deux à une concentration de 10⁻⁶ M). L'activité des composés selon l'invention (pKa) est supérieure ou égale à 6.0 (elle est en général comprise entre 6.0 et 7.6). Leur efficacité est supérieure ou égale à 60% et se situe en général dans une gamme de 60 à 90%.

Les activités des composés selon l'invention envers les récepteurs bêta-1 et bêta-2 ont été étudiées sur l'oreillette et sur la trachée, respectivement, de cochons d'Inde. Les activités agonistes et antagonistes ont été mesurées. On a ainsi trouvé que les composés selon l'invention sont sélectifs envers les récepteurs bêta-3 : en effet, ils sont au moins 50 fois plus actifs envers les récepteurs bêta-3 qu'envers les récepteurs bêta-1 ou bêta-2.

Les composés selon l'invention peuvent donc être utilisés pour la préparation de médicaments, destinés notamment au traitement des maladies dans lesquelles les récepteurs bêta-3 sont impliqués. Plus particulièrement, les composés selon l'invention peuvent être utilisés comme médicaments à action bêta-3 agoniste.

Ainsi, selon un autre de ses aspects, l'invention a pour objet un médicament qui comprend un composé de formule (I), ou un sel d'addition de ce dernier à un acide pharmaceutiquement acceptable, ou encore un hydrate ou un solvat du composé de formule (I).

Des exemples de maladies dans lesquelles les récepteurs bêta-3 sont impliqués sont abondamment décrites dans littérature. Les composés de formule (I), ainsi que leurs sels pharmaceutiquement acceptables, ou hydrates ou solvats de ces composés, peuvent donc être indiqués pour le traitement des maladies gastro-intestinales, telles que les maladies inflammatoires de l'intestin, par exemple le syndrome du côlon irritable (IBS) ou la maladie du côlon inflammatoire (IBD), comme modulateurs de la motricité intestinale, comme lipolytiques, agents anti-obésité, anti-diabétiques, anti-glaucomateux, cicatrisants, comme inhibiteurs des contractions utérines, comme tocolytiques pour prévenir ou retarder les accouchements précoces, ainsi que pour le traitement et/ou la prophylaxie de la dysménorrhée. En outre, les composés de formule (I), ainsi que leurs sels pharmaceutiquement acceptables, ou hydrates ou solvats de ces composés, peuvent être utilisés dans le traitement de certaines maladies du système nerveux central, par exemple comme psychotropes ou anti-dépresseurs, ainsi que de certains troubles du système urinaire, tel que l'incontinence urinaire.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables, ou un hydrate ou solvat de ce composé.

La présente invention concerne également des compositions pharmaceutiques comprenant, en tant que principe actif, au moins un composé selon l'invention. Ces compositions pharmaceutiques contiennent une dose efficace d'un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

La dose de principe actif administrée par se situe entre 0,01 et 20 mg par kilo de poids corporel du mammifère à traiter, de préférence entre 0,1 et 10 mg/kg. Chez l'être humain, la dose peut varier de 0,5 mg à 1500 mg par jour, par exemple de 2,5 à 500 mg selon l'âge du sujet à traiter, le type de traitement (prophylactique ou curatif) et la gravité de l'affection. Les composés de formule (I) sont généralement administrés en forme unitaire de dosage de 0,1 à 500 mg, de préférence de 0,5 à 100 mg de principe actif, entre une et cinq fois par jour.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications (Revendications pour l'(les) Etat(s) contractant(s) suivant(s): BG, CZ, EE, HU, RO, SI, SK)

1. Composés répondant à la formule (I) 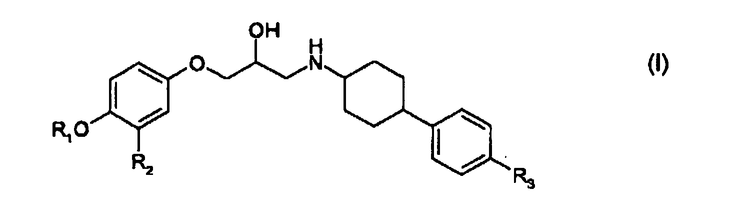 dans laquelle :
R₁ représente un atome d'hydrogène ou un groupe (C1-C4)alkyle, un groupe -CO(C1-C4)alkyle, un groupe (C1-C4)alkyl-phényle ou un groupe -CO-phényle, ledit phényle étant éventuellement substitué par un à trois groupes choisis indépendamment les uns des autres parmi les atomes d'halogène, les groupes (C1-C4)alkyles et (C1-C4)alcoxy ;
R₂ est choisi parmi l'un des groupes suivants :
. un atome d'hydrogène,
. un atome d'halogène,
. un groupe -S(O)_{z}(C1-C4)alkyle, où z est égal à 0, 1 ou 2,
. un groupe -NHSO₂(C1-C4)alkyle,
. un groupe -NHSO₂-phényle, ou
. un groupe -NHSO₂-(C1-C4)alkyl-phényle,
où ledit phényle est éventuellement substitué par un à trois groupes choisis indépendamment les uns des autres parmi les atomes d'halogène, les groupes (C1-C4)alkyles et (C1-C4)alcoxy ; et
R₃ est choisi parmi l'un des groupes suivants :
• un groupe -X-R₄, dans lequel X représente une liaison, un atome d'oxygène ou un groupe -CH₂- et R₄ représente un atome d'hydrogène ou un groupe de formule (C1-C4)alkyles et (C1-C4)alcoxy, ou fusionné avec un groupe dioxolane, ou
• un groupe -CO-NR₈R₉, dans lequel R₈ représente un atome d'hydrogène, un groupe (C1-C4)alkyle ou un groupe (C1-C4)alkyl-(C1-C4)alcoxy et R₉ est choisi parmi l'un des groupes suivants :
- un groupe (C1-C4)alkyl-(C1-C4)alcoxy,
- un groupe de formule -(CH₂)ₙ-A, où n est égal à 0, 1, 2, 3 ou 4 et où A représente un groupe indolyle, fluorène ou un groupe phényle, où le groupe phényle est substitué par un à trois groupes choisis indépendamment parmi les atomes d'halogène et les groupes hydroxy ou (C1-C4)alkyles,
- un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
- un groupe de formule -CH(R₁₀)-(CH₂)ₙ-COOR₁₁, où n est égal à 0, 1, 2 ou 3, R₁₁ représente un atome d'hydrogène ou un groupe (C1-C4)alkyle, et R₁₀ représente :
. un atome d'hydrogène,
. un groupe (C1-C4)alkyle,
. un groupe -COOR₁₂, où R₁₂ représente un atome d'hydrogène ou un groupe (C1-C4)alkyle, ou
. un groupe -CH₂-phényle, où le groupe phényle est éventuellement substitué par un à trois groupes choisis indépendamment les uns des autres parmi les atomes d'halogène et les groupes hydroxy ou (C1-C4)alkyles ;
à l'état de bases ou de sels d'addition à des acides, ainsi qu'à l'état d'hydrates ou de solvats à l'exclusion des composés pour lesquels R3 représente un groupe X-R4, X représentant un groupe -CH₂- et R4 représentant un hydrogène lorsque R1 représente un atome d'hydrogène, un groupe (C1-C4)alkyle, un groupe -CO(C1-C4)alkyle ou un groupe -CO-phényle, ledit phényle étant éventuellement substitué par un groupe choisi parmi un atome d'halogène, un groupe (C1-C4)alkyles et (C1-C4)alcoxy ; et
R₂ est choisi parmi l'un des groupes suivants :
. un atome d'hydrogène,
. un atome d'halogène,
. un groupe -S(O)_{z}(C1-C4)alkyle, où z est égal à 0.1 ou 2,
. un groupe -NHSO₂(C1-C4)alkyle, ou
. un groupe -NHSO₂-phényle,
où ledit phényle est éventuellement substitué par un groupe choisi parmi un atome d'halogène, un groupe (C1-C4)alkyle et (C1-C4)alcoxy.

2. Composé selon la revendication 1 **caractérisé en ce que** :
R₃ est choisi parmi l'un des groupes suivants :
• un groupe -X-R₄, dans lequel X représente une liaison ou un groupe -CH₂- et R₄ représente un groupe de formule -CR₅R₆-COOR₇; ou X représente un atome d'oxygène et R₄ représente un atome d'hydrogène ou un groupe de formule -CR₅R₆-COOR₇, où R₅, R₆ et R₇ représentent indépendamment les uns des autres un atome d'hydrogène ou un groupe (C1-C4)alkyle,
• un groupe phényle éventuellement substitué par un à trois groupes choisis indépendamment les uns des autres parmi les atomes d'halogène, les groupes (C₁-C₄)alkyles et (C1-C4)alcoxy, ou fusionné avec un groupe dioxolane, ou
• un groupe -CO-NR₈R₉, dans lequel R₈ représente un atome d'hydrogène, un groupe (C1-C4)alkyle ou un groupe (C1-C4)alkyl-(C1-C4)alcoxy et R₉ est choisi parmi l'un des groupes suivants :
- un groupe (C1-C4)alkyl-(C1-C4)alcoxy,
- un groupe de formule -(CH₂)ₙ-A, où n est égal à 0, 1, 2, 3 ou 4 et où A représente un groupe indolyle, fluorène ou un groupe phényle, où le groupe phényle est substitué par un à trois groupes choisis indépendamment parmi les atomes d'halogène et les groupes hydroxy ou (C1-C4)alkyles,
- un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
- un groupe de formule -CH(R₁₀)-(CH₂)ₙ-COOR₁₁, où n est égal à 0, 1, 2 ou 3, R₁₁ représente un atome d'hydrogène ou un groupe (C1-C4)alkyle, et R₁₀ représente :
. un atome d'hydrogène,
. un groupe (C1-C4)alkyle,
. un groupe -COOR₁₂, où R₁₂ représente un atome d'hydrogène ou un groupe (C1-C4)alkyle, ou
. un groupe -CH₂-phényle, où le groupe phényle est éventuellement substitué par un à trois groupes choisis indépendamment les uns des autres parmi les atomes d'halogène et les groupes hydroxy ou (C1-C4)alkyles;
à l'état de bases ou de sels d'addition à des acides, ainsi qu'à l'état d'hydrates ou de solvats.

3. Composés de formule (I) selon la revendication 1 ou 2, **caractérisés en ce que** R₁ représente un atome d'hydrogène,
à l'état de bases ou de sels d'addition à des acides, ainsi qu'à l'état d'hydrates ou de solvats.

4. Composés de formule (I) selon la revendication 1, 2 ou la revendication 3, **caractérisés en ce que** R₂ représente un groupe -SO₂(C1-C4)alkyle ou -NHSO₂(C1-C4)alkyle,
à l'état de bases ou de sels d'addition à des acides, ainsi qu'à l'état d'hydrates ou de solvats.

5. Composés selon l'une quelconque des revendications 1, 3 ou 4, **caractérisés en ce que** R₃ est choisi parmi l'un des groupes suivants :
• un groupe -X-R₄, dans lequel X et R₄ sont tels que définis dans la revendication 1, R₄ ne représentant toutefois pas un atome d'hydrogène lorsque X représente une liaison ou un groupe -CH₂-,
• un groupe phényle éventuellement substitué par un à trois groupes choisis indépendamment les uns des autres parmi les atomes d'halogène, les groupes (C1-C4)alkyles et (C1-C4)alcoxy, ou fusionné avec un groupe dioxolane, ou
• un groupe -CO-NR₈R₉, dans lequel R₈ est tel que défini dans la revendication 1 et R₉ est choisi parmi l'un des groupes suivants :
- un group-(C1-C4)alkyl-(C1-C4)alcoxy,
- un groupe de formule -(CH₂)ₙ-A, où n est égal à 0, 1, 2 ,3 ou 4 et où A représente un groupe indolyle, un groupe fluorène ou un groupe phényle substitué par un à trois groupes choisis indépendamment parmi les atomes d'halogène et les groupes hydroxy ou (C1-C4)alkyles,
- un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
- un groupe de formule -CH(R₁₀)-(CH₂)ₙ-COOR₁₁, où n est égal à 0, 1, 2 ou 3 et où R₁₀ et R₁₁ sont tels que définis dans la revendication 1 ;
à l'état de bases ou de sels d'addition à des acides, ainsi qu'à l'état d'hydrates ou de solvats.

6. Composés selon la revendication 5, **caractérisés en ce que** R₃ représente un groupe -CO-NHR₉, dans lequel R₉ est choisi parmi l'un des groupes suivants :
- un groupe de formule -(CH₂)ₙ-A, où n est égal à 0. 1, 2, 3 ou 4 et où A représente un groupe indolyle, un groupe fluorène ou un groupe phényle substitué par un à trois groupes choisis indépendamment parmi les atomes d'halogène et les groupes hydroxy ou (C₁-C₄)alkyles.
- un groupe -NH-phényle où le groupe phényle est éventuellement substitué par un à trois groupes choisis indépendamment les uns des autres parmi les atomes d'halogène et les groupes hydroxy ou (C₁-C₄)alkyles, ou
- un groupe de formule -CH(R₁₀)-(CH₂)ₙ-COOR₁₁, où n est égal à 0, 1, 2 ou 3 et où R₁₀ et R₁₁ sont tels que définis dans la revendication 1;
à l'état de bases ou de sels d'addition à des acides, ainsi qu'à l'état d'hydrates ou de solvats.

7. Procédé de préparation des composés de formule (I) selon l'une quelconque des revendications 1 à 4, dans lesquels Rₛ est différent d'un groupe -CO-NR₈R₉, **caractérisé en ce que** l'on fait réagir un composé de formule (II), où R représente un groupe électrophile et Pg un groupe protecteur, avec un époxyde de formule (III), où R₁ et R₂ sont tels que définis dans l'une quelconque des revendications 1 à 4 : 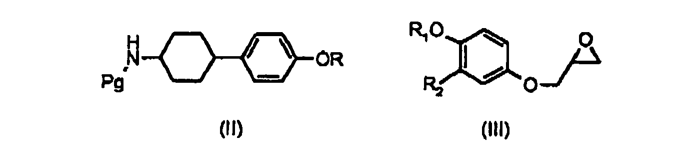 pour obtenir un composé de formule (IV) : 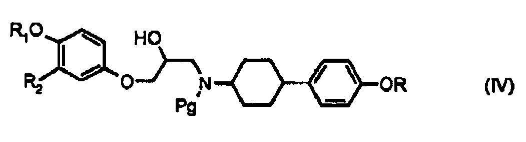 que l'on transforme en composé de formule (V), dans lequel R₃ est tel que défini dans l'une quelconque des revendications 1 à 4, mais est différent d'un groupe -CO-NR₈R₉ : 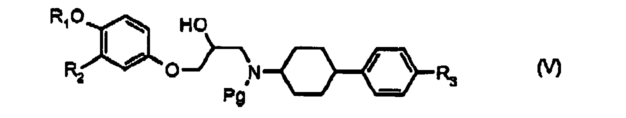 duquel on élimine ensuite les groupes protecteurs.

8. Procédé de préparation des composés de formule (1) selon l'une quelconque des revendications 1 à 6, dans lesquels R₃ représente un groupe -CO-NR₈R₉, **caractérisé en ce que** l'on fait réagir un composé de formule (XII) : 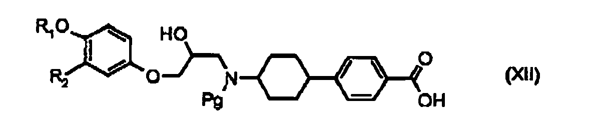 où R₁ et R₂ sont tels que définis dans l'une quelconque des revendications 1 à 4 et Pg représente un groupe protecteur, avec une amine de formule HNR₈R₉, dans laquelle R₈ et R₉ sont tels que définis dans l'une quelconque des revendications 1 à 6, puis que l'on élimine les groupes protecteurs du produit ainsi obtenu.

9. Médicament, **caractérisé en ce qu'**il comprend un composé de formule (I) selon l'une quelconque des revendications 1 à 6, ou un sel d'addition de ce composé à un acide pharmaceutiquement acceptable, ou encore un hydrate ou un solvat du composé de formule (I).

10. Composition pharmaceutique, **caractérisée en ce qu'**elle comprend un composé de formule (I) selon l'une quelconque des revendications 1 à 6, ou un sel pharmaceutiquement acceptable, un hydrate ou un solvat de ce composé, ainsi qu'au moins un excipient pharmaceutiquement acceptable.

11. Utilisation d'un composé de formule (1) selon l'une quelconque des revendications 1 à 6 pour la préparation d'un médicament destiné au traitement des maladies gastro-intestinales, comme modulateurs de la motricité intestinale, comme lipolytiques, agents anti-obésité, anti-diabétiques, anti-glaucomateux, cicatrisants, comme inhibiteurs des contractions utérines, comme tocolytiques pour prévenir ou retarder les accouchements précoces, pour le traitement et/ou la prophylaxie de la dysménorrhée, comme psychotropes ou anti-dépresseurs, ainsi que pour le traitement de l'incontinence urinaire.

## Revendications (Revendications pour l'(les) Etat(s) contractant(s) suivant(s): AT, BE, CH, CY, DE, DK, ES, FI, FR, GB, GR, IE, IT, LI, LU, MC, NL, PT, SE, TR)

1. Composé répondant à la formule (I) 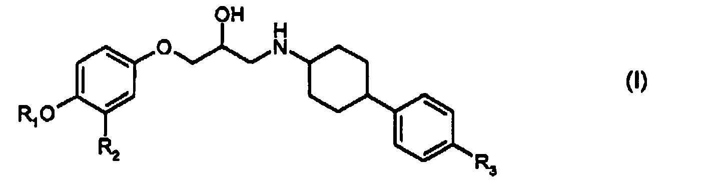 dans laquelle :
**R₁** représente un atome d'hydrogène ou un groupe (C1-Ca)alkyle, un groupe -CO(C1-C4)alkyle, un groupe (C1-C4)alkyl-phényle ou un groupe -CO-phényle, ledit phényle étant éventuellement substitué par un à trois groupes choisis indépendamment les uns des autres parmi les atomes d'halogène, les groupes (C1-C4)alkyles et (C1-C4)alcoxy ;
**R₂** est choisi parmi l'un des groupes suivants :
. un atome d'hydrogène,
. un atome d'halogène,
. un groupe -S(O)_{z}(C1-C4)alkyle, où z est égal à 0, 1 ou 2,
. un groupe -NHSO₂(C1-C4)alkyle,
. un groupe -NHSO₂-phényle, ou
. un groupe -NHSO₂(C1-C4)alkyl-phényle,
où ledit phényle est éventuellement substitué par un à trois groupes choisis indépendamment les uns des autres parmi les atomes d'halogène, les groupes (C1-C4)alkyles et (C1-C4)alcoxy ; et
R₃ est choisi parmi l'un des groupes suivants :
• un groupe -X-R₄, dans lequel X représente une liaison, un atome d'oxygène ou un groupe -CH₂- et R₄ représente un atome d'hydrogène ou un groupe de formule -CR₅R₆-COOR₇, où R₅, R₆ et R₇ représentent indépendamment les uns des autres un atome d'hydrogène ou un groupe (C1-C4)alkyle, R₄ ne représentant toutefois pas un atome d'hydrogène lorsque X représente une liaison,
• un groupe phényle éventuellement substitué par un à trois groupes choisis indépendamment les uns des autres parmi les atomes d'halogène, les groupes (C1-C4)alkyles et (C1-C4)alcoxy, ou fusionné avec un groupe dioxolane, ou
• un groupe -CO-NR₈R₉, dans lequel R₈ représente un atome d'hydrogène, un groupe (C1-C4)alkyle ou un groupe (C1-C4)alkyl-(C1-C4)alcoxy et R₉ est choisi parmi l'un des groupes suivants :
- un groupe(C1-C4)alkyl-(C1-C4)alcoxy.
- un groupe de formule -(CH₂)ₙ-A, où n est égal à 0, 1, 2, 3 ou 4 et où A représente un groupe indolyle, fluorène ou un groupe phényle, où le groupe phényle est substitué par un à trois groupes choisis indépendamment parmi les atomes d'halogène et les groupes hydroxy ou (C1-C4)alkyles,
- un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
- un groupe de formule -CH(R₁₀)-(CH₂)ₙ-COOR₁₁, où n est égal à 0, 1, 2 ou 3, R₁₁ représente un atome d'hydrogène ou un groupe (C1-C4)alkyle, et R₁₀ représente :
. un atome d'hydrogène,
. un groupe (C1-C4)alkyle,
. un groupe -COOR₁₂, où R₁₂ représente un atome d'hydrogène ou un groupe (C1-C4)alkyle, ou
. un groupe -CH₂-phényle, où le groupe phényle est éventuellement substitué par un à trois groupes choisis indépendamment les uns des autres parmi les atomes d'halogène et les groupes hydroxy ou (C1-C4)alkyles ;
à l'état de bases ou de sels d'addition à des acides, ainsi qu'à l'état d'hydrates ou de solvats à l'exclusion des composés pour lesquels R3 représente un groupe X-R4, X représentant un groupe -CH₂ - et R4 représentant un hydrogène lorsque R1 représente un atome d'hydrogène, un groupe (C1-C4)alkyle, un groupe -CO(C1-C4)alkyle ou un groupe -CO-phényle, ledit phényle étant éventuellement substitué par un groupe choisi parmi un atome d'halogène, un groupe (C1-C4)alkyles et (C1-C4)alcoxy ; et
R₂ est choisi parmi l'un des groupes suivants :
. un atome d'hydrogène,
. un atome d'halogène,
. un groupe -S(O)_{z}(C1-C4)alkyle, où z est égal à 0,1 ou 2,
. un groupe -NHSO₂(C1-C4)alkyle, ou
. un groupe -NHSO₂-phényle,
où ledit phényle est éventuellement substitué par un groupe choisi parmi un atome d'halogène, un groupe (C1-C4)alkyle et (C1-C4)alcoxy.

2. Composé selon la revendication 1 **caractérisé en ce que** :
R₃ est choisi parmi l'un des groupes suivants :
• un groupe -X-R₄, dans lequel X représente une liaison ou un groupe -CH₂- et R₄ représente un groupe de formule -CR₅R₆-COOR₇; ou X représente un atome d'oxygène et R₄ représente un atome d'hydrogène ou un groupe de formule -CR₅R₆-COOR₇, où R₅, R₆ et R₇ représentent indépendamment les uns des autres un atome d'hydrogène ou un groupe (C1-C4)alkyle,
• un groupe phényle éventuellement substitué par un à trois groupes choisis indépendamment les uns des autres parmi les atomes d'halogène, les groupes (C₁-C₄)alkyles et (C₁-C₄)alcoxy, ou fusionné avec un groupe dioxolane, ou
• un groupe -CO-NR₈R₉, dans lequel R₈ représente un atome d'hydrogène, un groupe (C₁-C₄)alkyle ou un groupe (C₁-C₄)alkyl-(C₁-C₄)alcoxy et R₉ est choisi parmi l'un des groupes suivants :
- un groupe (C₁-C₄)alkyl-(C₁-C₄)alcoxy,
- un groupe de formule -(CH₂)ₙ-A, où n est égal à 0, 1, 2, 3 ou 4 et où A représente un groupe indolyle, fluorène ou un groupe phényle, où le groupe phényle est substitué par un à trois groupes choisis indépendamment parmi les atomes d'halogène et les groupes hydroxy ou (C₁-C₄)alkyles,
- un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
- un groupe de formule -CH(R₁₀)-(CH₂)ₙ-COOR₁₁, où n est égal à 0, 1, 2 ou 3, R₁₁ représente un atome d'hydrogène ou un groupe (C₁-C₄)alkyle, et R₁₀ représente :
. un atome d'hydrogène,
. un groupe (C1-C4)alkyle,
. un groupe -COOR₁₂, où R₁₂ représente un atome d'hydrogène ou un groupe (C1-C4)alkyle, ou
. un groupe -CH₂-phényle, où le groupe phényle est éventuellement substitué par un à trois groupes choisis indépendamment les uns des autres parmi les atomes d'halogène et les groupes hydroxy ou (C₁-C₄)alkyles;
à l'état de bases ou de sels d'addition à des acides, ainsi qu'à l'état d'hydrates ou de solvats.

3. Composés de formule (I) selon la revendication 1 ou 2, **caractérisés en ce que** R₁ représente un atome d'hydrogène,
à l'état de bases ou de sels d'addition à des acides, ainsi qu'à l'état d'hydrates ou de solvats.

4. Composés de formule (I) selon la revendication 1,2 ou la revendication 3, **caractérisés en ce que** R₂ représente un groupe -SO₂(C1-C4) alkyle ou -NHSO₂(C1-C4)alkyle, à l'état de bases ou de sels d'addition à des acides, ainsi qu'à l'état d'hydrates ou de solvats.

5. Composés selon l'une quelconque des revendications 1 à 4, **caractérisés en ce que** R₃ est choisi parmi l'un des groupes suivants :
• un groupe -X-R₄, dans lequel X et R₄ sont tels que définis dans la revendication 1 ou 2 ;
• un groupe phényle éventuellement substitué par un à trois groupes choisis indépendamment les uns des autres parmi les atomes d'halogène, les groupes (C1-C4)alkyles et (C1-C4)alcoxy, ou fusionné avec un groupe dioxolane, ou
• un groupe -CO-NR₈R₉, dans lequel R₈ est tel que défini dans la revendication 1 et R₉ est choisi parmi l'un des groupes suivants :
- un groupe-(C1-C4)alkyl-(C1-C4)alcoxy,
- un groupe de formule -(CH₂)ₙ-A, où n est égal à 0, 1, 2 ,3 ou 4 et où A représente un groupe indolyle, un groupe fluorène ou un groupe phényle substitué par un à trois groupes choisis indépendamment parmi les atomes d'halogène et les groupes hydroxy ou (C1-C4)alkyles,
- un groupe -NH-phényle, où le groupe phényle est éventuellement substitué par un à trois groupes choisis indépendamment les uns des autres parmi les atomes d'halogène et les groupes hydroxy ou (C1-C4)alkyles, ou
- un groupe de formule -CH(R₁₀)-(CH₂)ₙ-COOR₁₁, où n est égal à 0, 1, 2 ou 3 et où R₁₀ et R₁₁ sont tels que définis dans la revendication 1 ;
à l'état de bases ou de sels d'addition à des acides, ainsi qu'à l'état d'hydrates ou de solvats.

6. Composés selon la revendication 5, **caractérisés en ce que** R₃ représente un groupe -CO-NHR₉, dans lequel R₉ est choisi parmi l'un des groupes suivants :
- un groupe de formule -(CH₂)ₙ-A, où n est égal à 0. 1, 2, 3 ou 4 et où A représente un groupe indolyte, un groupe fluorène ou un groupe phényle substitué par un à trois groupes choisis indépendamment parmi les atomes d'halogène et les groupes hydroxy ou (C1-C4)alkyles,
- un groupe -NH-phényle où le groupe phényle est éventuellement substitué par un à trois groupes choisis indépendamment les uns des autres parmi les atomes d'halogène et les groupes hydroxy ou (C₁-C₄)alkyles, ou
- un groupe de formule -CH(R₁₀)-(CH₂)ₙ-COOR₁₁, où n est égal à 0, 1, 2 ou 3 et où R₁₀ et R₁₁ sont tels que définis dans la revendication 1 ;
à l'état de bases ou de sels d'addition à des acides, ainsi qu'à l'état d'hydrates ou de solvats.

7. Procédé de préparation des composés de formule (I) selon l'une quelconque des revendications 1 à 4, dans lesquels R₃ est différent d'un groupe -CO-NR₈R₉, **caractérisé en ce que** l'on fait réagir un composé de formule (II), où R représente un groupe électrophile et Pg un groupe protecteur, avec un époxyde de formule (III), où R₁ et R₂ sont tels que définis dans l'une quelconque des revendications 1 à 4 : 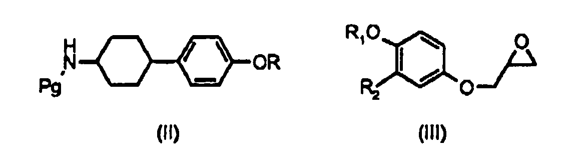 pour obtenir un composé de formule (IV) : 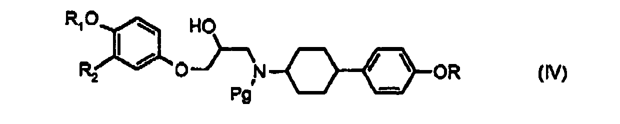 que l'on transforme en composé de formule (V), dans lequel R₃ est tel que défini dans l'une quelconque des revendications 1 à 5, mais est différent d'un groupe -CO-NR₈R₉ : 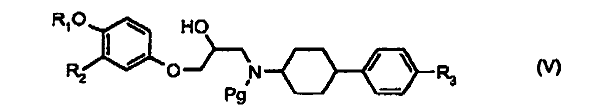 duquel on élimine ensuite les groupes protecteurs.

8. Procédé de préparation des composés de formule (I) selon l'une quelconque des revendications 1 à 6, dans lesquels R₃ représente un groupe -CO-NR₈R₉, **caractérisé en ce que** l'on fait réagir un composé de formule (XII) : 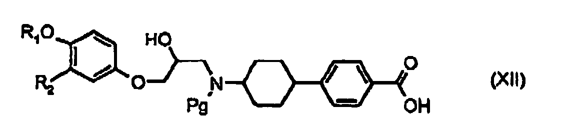 où R₁ et R₂ sont tels que définis dans l'une quelconque des revendications 1 à 4 et Pg représente un groupe protecteur, avec une amine de formule HNR₈R₉, dans laquelle R₈ et R₉ sont tels que définis dans l'une quelconque des revendications 1 à 6, puis que l'on élimine les groupes protecteurs du produit ainsi obtenu.

9. Médicament, **caractérisé en ce qu'**il comprend un composé de formule (I) selon l'une quelconque des revendications 1 à 6, ou un sel d'addition de ce composé à un acide pharmaceutiquement acceptable, ou encore un hydrate ou un solvat du composé de formule (I).

10. Composition pharmaceutique, **caractérisée en ce qu'**elle comprend un composé de formule (I) selon l'une quelconque des revendications 1 à 6, ou un sel pharmaceutiquement acceptable, un hydrate ou un solvat de ce composé, ainsi qu'au moins un excipient pharmaceutiquement acceptable.

11. Utilisation d'un composé de formule (I) selon l'une quelconque des revendications 1 à 6 pour la préparation d'un médicament destiné au traitement des maladies gastro-intestinales, comme modulateurs de la motricité intestinale, comme lipolytiques, agents anti-obésité, anti-diabétiques, anti-glaucomateux, cicatrisants, comme inhibiteurs des contractions utérines, comme tocolytiques pour prévenir ou retarder les accouchements précoces, pour le traitement et/ou la prophylaxie de la dysménorrhée, comme psychotropes ou anti-dépresseurs, ainsi que pour le traitement de l'incontinence urinaire.

## Claims (Claims for the following Contracting State(s): BG, CZ, EE, HU, RO, SI, SK)

1. Compounds corresponding to the formula (I) 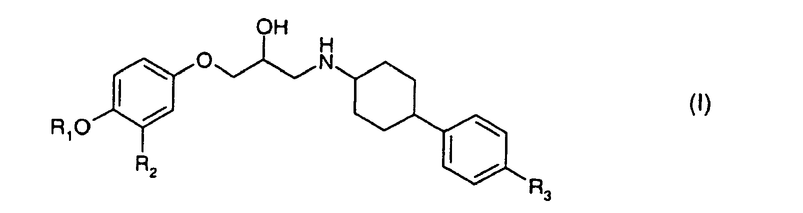 in which:
R₁ represents a hydrogen atom or a (C₁-C₄)alkyl group, a -CO(C₁-C₄)alkyl group, a (C₁-C₄) alkylphenyl group or a -CO-phenyl group, said phenyl optionally being substituted by one to three groups chosen, independently of one another, from halogen atoms, (C₁-C₄)alkyl groups and (C₁-C₄) alkoxy groups;
R₂ is chosen from one of the following groups:
. a hydrogen atom,
. a halogen atom,
. an -S(O)_{z}(C₁-C₄)alkyl group, where z is equal to 0, 1 or 2,
. an -NHSO₂(C₁-C₄)alkyl group,
. an -NHSO₂-phenyl group, or
. an -NHSO₂-(C₁-C₄)alkylphenyl group,
where said phenyl is optionally substituted by one to three groups chosen, independently of one another, from halogen atoms, (C₁-C₄) alkyl groups and (C₁-C₄) alkoxy groups; and
R₃ is chosen from one of the following groups:
• an -X-R₄ group, in which X represents a bond, an oxygen atom or a -CH₂- group and R₄ represents a hydrogen atom or a group of formula -CR₅R₆-COOR₇, where R₅, R₆ and R₇ represent, independently of one another, a hydrogen atom or a (C₁-C₄) alkyl group, R₄ not representing, however, a hydrogen atom when X represents a bond,
• a phenyl group optionally substituted by one to three groups chosen, independently of one another, from halogen atoms, (C₁-C₄) alkyl groups and (C₁-C₄)alkoxy groups or fused with a dioxolane group, or
• a -CO-NR₈R₉ group, in which R₈ represents a hydrogen atom, a (C₁-C₄) alkyl group or a (C₁-C₄) alkoxy (C₁-C₄) alkyl group and R₉ is chosen from one of the following groups:
- a (C₁-C₄) alkoxy (C₁-C₄) alkyl group,
- a group of formula (CH₂)ₙ-A, where n is equal to 0, 1, 2, 3 or 4 and where A represents an indolyl, fluorenyl or phenyl group, where the phenyl group is substituted by one to three groups chosen, independently of one another, from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3, R₁₁ represents a hydrogen atom or a (C₁-C₄) alkyl group and R₁₀ represents:
. a hydrogen atom,
. a (C₁-C₄) alkyl group,
. a -COOR₁₂ group, where R₁₂ represents a hydrogen atom or a (C₁-C₄)alkyl group, or
. a -CH₂-phenyl group, where the phenyl group is optionally substituted by one to three groups chosen, independently of one another, from halogen atoms, hydroxyl groups and (C₁-C₄)alkyl groups;
in the form of bases or of addition salts with acids, and in the form of hydrates or of solvates.

2. Compounds according to Claim 1, **characterized in that**:
R₃ is chosen from one of the following groups:
• an -X-R₄ group, in which X represents a bond or a -CH₂- group and R₄ represents a group of formula -CR₅R₆-COOR₇; or X represents an oxygen atom and R₄ represents a hydrogen atom or a group of formula -CR₅R₆-COOR₇, where R₅, R₆ and R₇ represent, independently of one another, a hydrogen atom or a (C₁-C₄)alkyl group,
• a phenyl group optionally substituted by one to three groups chosen, independently of one another, from halogen atoms, (C₁-C₄) alkyl groups and (C₁-C₄)alkoxy groups or fused with a dioxolane group, or
• a -CO-NR₈R₉ group, in which R₈ represents a hydrogen atom, a (C₁-C₄) alkyl group or a (C₁-C₄)alkoxy(C₁-C₄)alkyl group and R₉ is chosen from one of the following groups:
- a (C₁-C₄) alkoxy(C₁-C₄) alkyl group,
- a group of formula -(CH₂)ₙ-A, where n is equal to 0, 1, 2, 3 or 4 and where A represents an indolyl, fluorenyl or phenyl group, where the phenyl group is substituted by one to three groups chosen, independently of one another, from halogen atoms, hydroxyl groups and (C₁-C₄)alkyl groups,
- an -NH-phenyl group, where the phenyl group is optionally substituted by one to three groups chosen, independently, from halogen atoms, hydroxyl groups and (C₁-C4)alkyl groups, or
- a group of formula -CH(R₁₀)-(CH₂)ₙ-COOR₁₁, where n is equal to 0, 1, 2 or 3, R₁₁ represents a hydrogen atom or a (C₁-C₄)alkyl group and R₁₀ represents:
. a hydrogen atom,
. a (C₁-C₄) alkyl group,
. a -COOR₁₂ group, where R₁₂ represents a hydrogen atom or a (C₁-C₄)alkyl group, or
. a -CH₂-phenyl group, where the phenyl group is optionally substituted by one to three groups chosen, independently of one another, from halogen atoms, hydroxyl groups and (C₁-C₄)alkyl groups;
in the form of bases or of addition salts with acids, and in the form of hydrates or of solvates.

3. Compounds of formula (I) according to Claim 1 or 2, **characterized in that** R₁ represents a hydrogen atom,
in the form of bases or of addition salts with acids, and in the form of hydrates or of solvates.

4. Compounds of formula (I) according to Claim 1, 2 or 3, **characterized in that** R₂ represents an -SO₂(C₁-C₄)alkyl group or an -NHSO₂(C₁-C₄)alkyl group,
in the form of bases or of addition salts with acids, and in the form of hydrates or of solvates.

5. Compounds according to any one of Claims 1, 3 and 4, **characterized in that** R₃ is chosen from one of the following groups:
• an -X-R₄ group, in which X and R₄ are as defined in Claim 1, R₄ not representing, however, a hydrogen atom when X represents a bond or a -CH₂- group,
• a phenyl group optionally substituted by one to three groups chosen, independently of one another, from halogen atoms, (C₁-C₄) alkyl groups and (C₁-C₄)alkoxy groups or fused with a dioxolane group, or
• a -CO-NR₈R₉ group, in which R₈ is as defined in claim 1 and R₉ is chosen from one of the following groups:
- a (C₁-C₄)alkoxy(C₁-C₄)alkyl group,
- a group of formula -(CH₂)ₙ-A, where n is equal to 0, 1, 2, 3 or 4 and where A represents an indolyl group, a fluorenyl group or a phenyl group substituted by one to three groups independently chosen from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3 and where R₁₀ and R₁₁ are as defined in claim 1;
in the form of bases or of addition salts with acids, and in the form of hydrates or of solvates.

6. Compounds according to Claim 5, **characterized in that** R₃ represents a -CO-NHR₉ group, in which R₉ is chosen from one of the following groups:
- a group of formula -(CH₂)ₙ-A, where n is equal to 0, 1, 2, 3 or 4 and where A represents an indolyl group, a fluorenyl group or a phenyl group substituted by one to three groups independently chosen from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3 and where R₁₀ and R₁₁ are as defined in Claim 1; in the form of bases or of addition salts with acids, and in the form of hydrates or of solvates.

7. Process for the preparation of the compounds of formula (I) according to any one of Claims 1 to 5, in which R₃ is other than a -CO-NR₈R₉ group, **characterized in that** a compound of formula (II), where R represents an electrophilic group and Pg represents a protective group, is reacted with an epoxide of formula (III),
where R₁ and R₂ are as defined in any one of Claims 1 to 3: 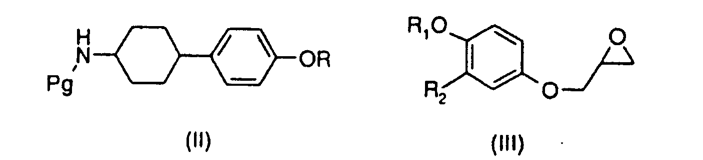 in order to obtain a compound of formula (IV): 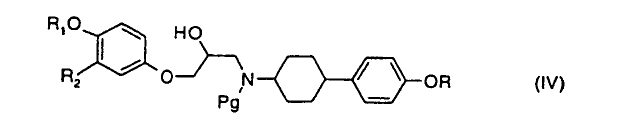 which is converted to a compound of formula (V), in which R₃ is as defined in any one of Claims 1 to 4 but is other than a -CO-NR₈R₉ group: 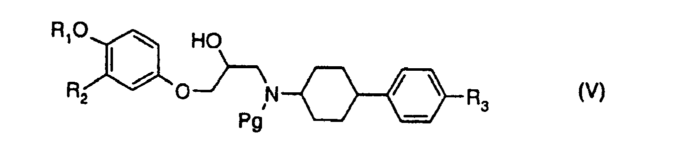 from which the protective groups are subsequently removed.

8. Process for the preparation of the compounds of formula (I) according to any one of Claims 1 to 6, in which R₃ represents a -CO-NR₈R₉ group, **characterized in that** the compound of formula (XII): 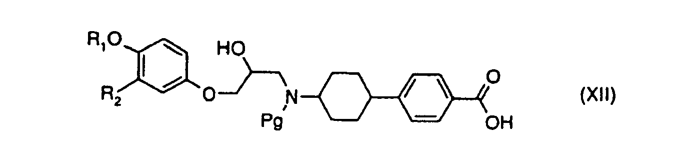 where R₁ and R₂ are as defined in any one of Claims 1 to 4 and Pg represents a protective group, is reacted with an amine of formula HNR₈R₉, in which R₈ and R₉ are as defined in any one of Claims 1 to 6, and then **in that** the protective groups are removed from the product thus obtained.

9. Medicament, **characterized in that** it comprises a compound of formula (I) according to any one of Claims 1 to 6, or an addition salt of this compound with a pharmaceutically acceptable acid, or also a hydrate or a solvate of the compound of formula (I).

10. Pharmaceutical composition, **characterized in that** it comprises a compound of formula (I) according to any one of Claims 1 to 6, or a pharmaceutically acceptable salt, a hydrate or a solvate of this compound, and at least one pharmaceutically acceptable excipient.

11. Use of a compound of formula (I) according to any one of Claims 1 to 6 in the preparation of a medicament intended for the treatment of gastrointestinal diseases, as modulators of intestinal motricity, as lipolytics, antiobesity agents, antidiabetics, antiglaucoma agents or cicatrizants, as inhibitors of uterine contractions, as tocolytics for preventing or delaying premature labour, for the treatment and/or prophylaxis of dysmenorrhoea, as psychotropics or antidepressants, and for the treatment of urinary incontinence.

## Claims (Claims for the following Contracting State(s): AT, BE, CH, CY, DE, DK, ES, FI, FR, GB, GR, IE, IT, LI, LU, MC, NL, PT, SE, TR)

1. Compounds corresponding to the formula (I) 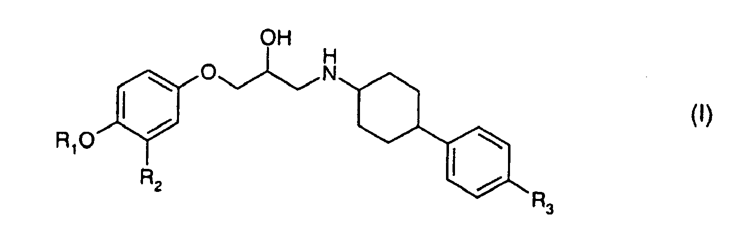 in which:
R₁ represents a hydrogen atom or a (C₁-C₄) alkyl group, a -CO(C₁-C₄)alkyl group, a (C₁-C₄) alkylphenyl group or a -CO-phenyl group, said phenyl optionally being substituted by one to three groups chosen, independently of one another, from halogen atoms, (C₁-C₄)alkyl groups and (C₁-C₄)alkoxy groups;
R₂ is chosen from one of the following groups:
. a hydrogen atom,
. a halogen atom,
. an -S (O)_{z} (C₁-C₄) alkyl group, where z is equal to 0, 1 or 2,
. an -NHSO₂(C₁-C₄)alkyl group,
. an -NHSO₂-phenyl group, or
. an -NHSO₂-(C₁-C₄)alkylphenyl group,
where said phenyl is optionally substituted by one to three groups chosen, independently of one another, from halogen atoms, (C₁-C₄)alkyl groups and (C₁-C₄)alkoxy groups; and
R₃ is chosen from one of the following groups:
• an -X-R₄ group, in which X represents a bond, an oxygen atom or a -CH₂- group and R₄ represents a hydrogen atom or a group of formula -CR₅R₆-COOR₇, where R₅, R₆ and R₇ represent, independently of one another, a hydrogen atom or a (C₁-C₄) alkyl group, R₄ not representing, however, a hydrogen atom when X represents a bond,
• a phenyl group optionally substituted by one to three groups chosen, independently of one another, from halogen atoms, (C₁-C₄)alkyl groups and (C₁-C₄)alkoxy groups or fused with a dioxolane group, or
• a -CO-NR₈R₉ group, in which R₈ represents a hydrogen atom, a (C₁-C₄) alkyl group or a (C₁-C₄)alkoxy(C₁-C₄)alkyl group and R₉ is chosen from one of the following groups:
- a (C₁-C₄) alkoxy (C₁-C₄) alkyl group,
- a group of formula -(CH₂)ₙ-A, where n is equal to 0, 1, 2, 3 or 4 and where A represents an indolyl, fluorenyl or phenyl group, where the phenyl group is substituted by one to three groups chosen, independently of one another, from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3, R₁₁ represents a hydrogen atom or a (C₁-C₄)alkyl group and R₁₀ represents:
. a hydrogen atom,
. a (C₁-C₄) alkyl group,
. a -COOR₁₂ group, where R₁₂ represents a hydrogen atom or a (C₁-C₄)alkyl group, or
. a -CH₂-phenyl group, where the phenyl group is optionally substituted by one to three groups chosen, independently of one another, from halogen atoms, hydroxyl groups and (C₁-C₄)alkyl groups;
in the form of bases or of addition salts with acids, and in the form of hydrates or of solvates, with the exclusion of the compounds for which R₃ represents an X-R₄ group, X representing a -CH₂- group and R₄ representing a hydrogen when R₁ represents a hydrogen atom, a (C₁-C₄) alkyl group, a -CO(C₁-C₄)alkyl group or a -CO-phenyl group, the said phenyl optionally being substituted by a group chosen from a halogen atom, a (C₁-C₄) alkyl group and a (C₁-C₄) alkoxy group; and
R₂ is chosen from one of the following groups:
. a hydrogen atom,
. a halogen atom,
. an -S(O)_{z}(C₁-C₄)alkyl group, where z is equal to 0, 1 or 2,
. an -NHSO₂(C₁-C₄)alkyl group, or
. an -NHSO₂-phenyl group,
where the said phenyl is optionally substituted by a group chosen from a halogen atom, a (C₁-C₄)alkyl group and a (C₁-C₄)alkoxy group.

2. Compounds according to Claim 1, **characterized in that**:
R₃ is chosen from one of the following groups:
• an -X-R₄ group, in which X represents a bond or a -CH₂- group and R₄ represents a group of formula -CR₅R₆-COOR₇; or X represents an oxygen atom and R₄ represents a hydrogen atom or a group of formula -CR₅R₆-COOR₇, where R₅, R₆ and R₇ represent, independently of one another, a hydrogen atom or a (C₁-C₄)alkyl group,
• a phenyl group optionally substituted by one to three groups chosen, independently of one another, from halogen atoms, (C₁-C₄) alkyl groups and (C₁-C₄) alkoxy groups or fused with a dioxolane group, or
• a -CO-NR₈R₉ group, in which R₈ represents a hydrogen atom, a (C₁-C₄) alkyl group or a (C₁-C₄)alkoxy(C₁-C₄)alkyl group and R₉ is chosen from one of the following groups:
- a (C₁-C₄)alkoxy(C₁-C₄)alkyl group,
- a group of formula -(CH₂)ₙ-A, where n is equal to 0, 1, 2, 3 or 4 and where A represents an indolyl, fluorenyl or phenyl group, where the phenyl group is substituted by one to three groups chosen, independently from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3, R₁₁ represents a hydrogen atom or a (C₁-C₄)alkyl group and R₁₀ represents:
. a hydrogen atom,
. a (C₁-C₄) alkyl group,
. a -COOR₁₂ group, where R₁₂ represents a hydrogen atom or a (C₁-C₄)alkyl group, or
. a -CH₂-phenyl group, where the phenyl group is optionally substituted by one to three groups chosen, independently of one another, from halogen atoms, hydroxyl groups and (C₁-C₄)alkyl groups; in the form of bases or of addition salts with acids, and in the form of hydrates or of solvates.

3. Compounds of formula (I) according to Claim 1 or 2, **characterized in that** R₁ represents a hydrogen atom,
in the form of bases or of addition salts with acids, and in the form of hydrates or of solvates.

4. Compounds of formula (I) according to Claim 1, 2 or Claim 3, **characterized in that** R₂ represents an -SO₂(C₁-C₄)alkyl group or an -NHSO₂(C₁-C₄)alkyl group,
in the form of bases or of addition salts with acids, and in the form of hydrates or of solvates.

5. Compounds according to any one of Claims 1 to 4, **characterized in that** R₃ is chosen from one of the following groups:
• an -X-R₄ group, in which X and R₄ are as defined in Claim 1 or 2;
• a phenyl group optionally substituted by one to three groups chosen, independently of one another, from halogen atoms, (C₁-C₄)alkyl groups and (C₁-C₄)alkoxy groups or fused with a dioxolane group, or
• a -CO-NR₈R₉ group, in which R₈ is as defined in claim 1 and R₉ is chosen from one of the following groups:
- a (C₁-C₄)alkoxy(C₁-C₄)alkyl group,
- a group of formula -(CH₂)ₙ-A, where n is equal to 0, 1, 2, 3 or 4 and where A represents an indolyl group, a fluorenyl group or a phenyl group substituted by one to three groups independently chosen from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3 and where R₁₀ and R₁₁ are as defined in claim 1; in the form of bases or of addition salts with acids, and in the form of hydrates or of solvates.

6. Compounds according to Claim 5, **characterized in that** R₃ represents a -CO-NHR₉ group, in which R₉ is chosen from one of the following groups:
- a group of formula -(CH₂)ₙ-A, where n is equal to 0, 1, 2, 3 or 4 and where A represents an indolyl group, a fluorenyl group or a phenyl group substituted by one to three groups independently chosen from halogen atoms, hydroxyl groups and (C₁-C₄)alkyl groups,
- an -NH-phenyl group, where the phenyl group is optionally substituted by one to three groups chosen, independently of one another, from halogen atoms, hydroxyl groups and (C₁-C₄)alkyl groups, or
- a group of formula -CH(R₁₀)-(CH₂)ₙ-COOR₁₁, where n is equal to 0, 1, 2 or 3 and where R₁₀ and R₁₁ are as defined in Claim 1; in the form of bases or of addition salts with acids, and in the form of hydrates or of solvates.

7. Process for the preparation of the compounds of formula (I) according to any one of Claims 1 to 4, in which R₃ is other than a -CO-NR₈R₉ group, **characterized in that** a compound of formula (II), where R represents an electrophilic group and Pg represents a protective group, is reacted with an epoxide of formula (III), where R₁ and R₂ are as defined in any one of Claims 1 to 4: 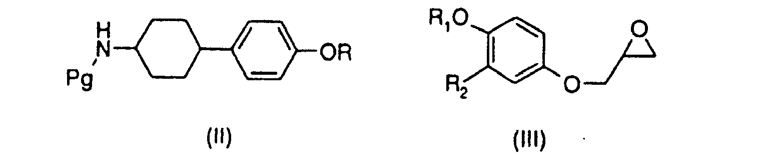 in order to obtain a compound of formula (IV): 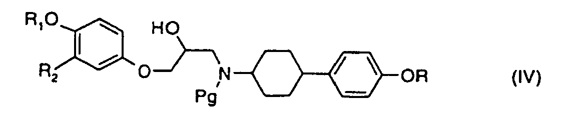 which is converted to a compound of formula (V), in which R₃ is as defined in any one of Claims 1 to 5 but is other than a -CO-NR₈R₉ group: 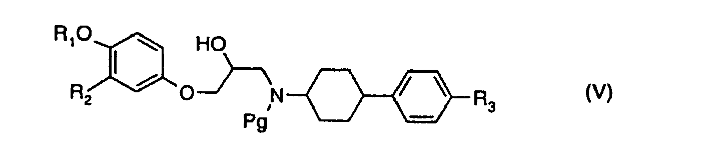 from which the protective groups are subsequently removed.

8. Process for the preparation of the compounds of formula (I) according to any one of Claims 1 to 6, in which R₃ represents a -CO-NR₈R₉ group, **characterized in that** the compound of formula (XII): 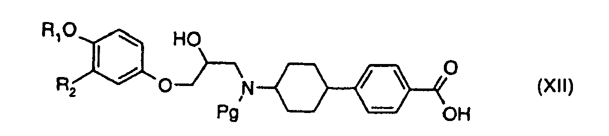 where R₁ and R₂ are as defined in any one of Claims 1 to 4 and Pg represents a protective group, is reacted with an amine of formula HNR₈R₉, in which R₈ and R₉ are as defined in any one of Claims 1 to 6, and then **in that** the protective groups are removed from the product thus obtained.

9. Medicament, **characterized in that** it comprises a compound of formula (I) according to any one of Claims 1 to 6, or an addition salt of this compound with a pharmaceutically acceptable acid, or also a hydrate or a solvate of the compound of formula (I).

10. Pharmaceutical composition, **characterized in that** it comprises a compound of formula (I) according to any one of Claims 1 to 6, or a pharmaceutically acceptable salt, a hydrate or a solvate of this compound, and at least one pharmaceutically acceptable excipient.

11. Use of a compound of formula (I) according to any one of Claims 1 to 6 in the preparation of a medicament intended for the treatment of gastrointestinal diseases, as modulators of intestinal motricity, as lipolytics, antiobesity agents, antidiabetics, antiglaucoma agents or cicatrizants, as inhibitors of uterine contractions, as tocolytics for preventing or delaying premature labour, for the treatment and/or prophylaxis of dysmenorrhoea, as psychotropics or antidepressants, and for the treatment of urinary incontinence.

## Patentansprüche (Patentansprüche für folgende(n) Vertragsstaat(en): BG, CZ, EE, HU, RO, SI, SK)

1. Verbindungen der Formel (I) 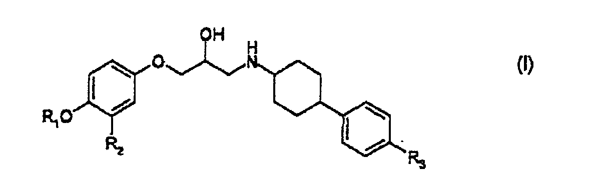 worin:
R₁ für ein Wasserstoffatom oder eine (C₁-C₄)-Alkylgruppe, eine -CO-(C₁-C₄)-Alkylgruppe, eine (C₁-C₄)-Alkylphenylgruppe oder eine -CO-Phenylgruppe steht, wobei das Phenyl gegebenenfalls durch eine bis drei unabhängig voneinander unter Halogenatomen, (C₁-C₄) -Alkylgruppen und (C₁-C₄) - Alkoxygruppen ausgewählte Gruppen substituiert ist;
R₂ unter den folgenden Gruppen ausgewählt ist:
. ein Wasserstoffatom,
. ein Halogenatom,
. eine -S (O)_{z}- (C₁-C₄) -Alkylgruppe, wobei z gleich 0, 1 oder 2 ist,
. eine -NHSO₂-(C₁-C₄)-Alkylgruppe,
. eine -NHSO₂-Phenylgruppe oder
. eine -NHSO₂-(C₁-C₄)-Alkylphenylgruppe,
wobei das Phenyl gegebenenfalls durch eine bis drei unabhängig voneinander unter Halogenatomen, (C₁-C₄) -Alkylgruppen und (C₁-C₄) -Alkoxygruppen ausgewählte Gruppen substituiert ist; und
R₃ unter den folgenden Gruppen ausgewählt ist:
• eine -X-R₄-Gruppe, worin X für eine Bindung, ein Sauerstoffatom oder eine -CH₂-Gruppe steht und R₄ für ein Wasserstoffatom oder eine Gruppe der Formel -CR₅R₆-COOR₇, wobei R₅, R₆ und R₇ unabhängig voneinander ein Wasserstoffatom oder eine (C₁-C₉)-Alkylgruppe bedeuten, steht, wobei R₄ jedoch nicht für ein Wasserstoffatom steht, wenn X für eine Bindung steht,
• eine Phenylgruppe, die gegebenenfalls durch eine bis drei unabhängig voneinander unter Halogenatomen, (C₁-C₄) -Alkylgruppen und (C₁-C₄) - Alkoxygruppen ausgewählte Gruppen substituiert oder mit einer Dioxolangruppe anelliert ist,
• eine -CO-NR₈R₉-Gruppe, worin R₈ für ein Wasserstoffatom, eine (C₁-C₄)-Alkylgruppe oder eine (C₁-C₄) -Alkoxy- (C₁-C₄) -alkylgruppe steht und R₉ unter den folgenden Gruppen ausgewählt ist:
- eine (C₁-C₄) -Alkoxy- (C₁-C₄) -alkylgruppe,
- eine Gruppe der Formel -(CH₂)ₙ-A, wobei n gleich 0, 1, 2, 3 oder 4 ist und A für eine Indolyl-, Fluoren- oder Phenylgruppe steht, wobei die Phenylgruppe durch eine bis drei unabhängig voneinander unter Halogenatomen und Hydroxy- oder (C₁-C₄)-Alkylgruppen ausgewählte Gruppen substituiert ist,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH(R₁₀)-(CH₂)ₙ-COOR₁₁, wobei n gleich 0, 1, 2 oder 3 ist, R₁₁ für ein Wasserstoffatom oder eine (C₁-C₄)-Alkylgruppe steht und R₁₀ für:
. ein Wasserstoffatom,
. eine (C₁-C₄)-Alkylgruppe,
. eine -COOR₁₂-Gruppe, wobei R₁₂ ein Wasserstoffatom oder eine (C₁-C₄)-Alkylgruppe bedeutet, oder
. eine -CH₂-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist,
steht;
in Form von Basen oder Säureadditionssalzen und in Form von Hydraten oder Solvaten.

2. Verbindungen nach Anspruch 1, **dadurch gekennzeichnet, daß**:
R₃ unter den folgenden Gruppen ausgewählt ist:
• eine -X-R₄-Gruppe, worin X für eine Bindung oder eine -CH₂-Gruppe steht und R₄ für eine Gruppe der Formel -CR₅R₆-COOR₇ steht; oder X für ein Sauerstoffatom steht und R₄ für ein Wasserstoffatom oder eine Gruppe der Formel -CR₅R₆-COOR₇ steht, wobei R₅, R₆ und R₇ unabhängig voneinander ein Wasserstoffatom oder eine (C₁-C₄)-Alkylgruppe bedeuten,
• eine Phenylgruppe, die gegebenenfalls durch eine bis drei unabhängig voneinander unter Halogenatomen, (C₁-C₄) -Alkylgruppen und (C₁-C₄)-Alkoxygruppen ausgewählte Gruppen substituiert oder mit einer Dioxolangruppe anelliert ist,
• eine -CO-NR₈R₉-Gruppe, worin R₈ für ein Wasserstoffatom, eine (C₁-C₄)-Alkylgruppe oder eine (C₁-C₄) -Alkoxy- (C₁-C₄) -alkylgruppe steht und R₉ unter den folgenden Gruppen ausgewählt ist:
- eine (C₁-C₄) -Alkoxy- (C₁-C₄) -alkylgruppe,
- eine Gruppe der Formel -(CH₂)ₙ-A, wobei n gleich 0, 1, 2, 3 oder 4 ist und A für eine Indolyl-, Fluoren- oder Phenylgruppe steht, wobei die Phenylgruppe durch eine bis drei unabhängig voneinander unter Halogenatomen und Hydroxy- oder (C₁-C₄)-Alkylgruppen ausgewählte Gruppen substituiert ist,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH(R₁₀)-(CH₂)ₙ-COOR₁₁, wobei n gleich 0, 1, 2 oder 3 ist, R₁₁ für ein Wasserstoffatom oder eine (C₁-C₄)-Alkylgruppe steht und R₁₀ für:
. ein Wasserstoffatom,
. eine (C₁-C₄)-Alkylgruppe,
. eine -COOR₁₂-Gruppe, wobei R₁₂ ein Wasserstoffatom oder eine (C₁-C₄)-Alkylgruppe bedeutet, oder
. eine -CH₂-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist,
steht;
in Form von Basen oder Säureadditionssalzen und in Form von Hydraten oder Solvaten.

3. Verbindungen der Formel (I) nach Anspruch 1 oder 2, **dadurch gekennzeichnet, daß** R₁ für ein Wasserstoffatom steht,
in Form von Basen oder Säureadditionssalzen und in Form von Hydraten oder Solvaten.

4. Verbindungen der Formel (I) nach Anspruch 1, 2 oder 3, **dadurch gekennzeichnet, daß** R₂ für eine -S(O)₂- (C₁-C₄) -Alkyl- oder -NHSO₂- (C₁-C₄) -Alkylgruppe steht,
in Form von Basen oder Säureadditionssalzen und in Form von Hydraten oder Solvaten.

5. Verbindungen nach einem der Ansprüche 1, 3 oder 4, **dadurch gekennzeichnet, daß** R₃ unter den folgenden Gruppen ausgewählt ist:
• eine -X-R₄-Gruppe, worin X und R₄ die in Anspruch 1 angegebene Bedeutung besitzen, wobei R₄ jedoch nicht für ein Wasserstoffatom steht, wenn X für eine Bindung oder eine -CH₂-Gruppe steht,
• eine Phenylgruppe, die gegebenenfalls durch eine bis drei unabhängig voneinander unter Halogenatomen, (C₁-C₄)-Alkylgruppen und (C₁-C₄)-Alkoxygruppen ausgewählte Gruppen substituiert oder mit einer Dioxolangruppe anelliert ist,
• eine -CO-NR₈R₉-Gruppe, worin R₈ die in Anspruch 1 angegebene Bedeutung besitzt und R₉ unter den folgenden Gruppen ausgewählt ist:
- eine (C₁-C₄)-Alkoxy-(C₁-C₄)-alkylgruppe,
- eine Gruppe der Formel -(CH₂)ₙ-A, wobei n gleich 0, 1, 2, 3 oder 4 ist und A für eine Indolylgruppe, eine Fluorengruppe oder eine Phenylgruppe, die durch eine bis drei unabhängig voneinander unter Halogenatomen und Hydroxy- oder (C₁-C₄)-Alkylgruppen ausgewählte Gruppen substituiert ist, steht,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH (R₁₀) - (CH₂)ₙ-COOR₁₁, wobei n gleich 0, 1, 2 oder 3 ist und R₁₀ und R₁₁ die in Anspruch 1 angegebene Bedeutung besitzen;
in Form von Basen oder Säureadditionssalzen und in Form von Hydraten oder Solvaten.

6. Verbindungen nach Anpruch 5, **dadurch gekennzeichnet, daß** R₃ für eine -CO-NHR₉-Gruppe steht, worin R₉ unter den folgenden Gruppen ausgewählt ist:
- eine Gruppe der Formel -(CH₂)ₙ-A, wobei n gleich 0, 1, 2, 3 oder 4 ist und A für eine Indolylgruppe, eine Fluorengruppe oder eine Phenylgruppe, die durch eine bis drei unabhängig voneinander unter Halogenatomen und Hydroxy- oder (C₁-C₄)-Alkylgruppen ausgewählte Gruppen substituiert ist, steht,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH (R₁₀) - (CH₂)ₙ-COOR₁₁, wobei n gleich 0, 1, 2 oder 3 ist und R₁₀ und R₁₁ die in Anspruch 1 angegebene Bedeutung besitzen;
in Form von Basen oder Säureadditionssalzen und in Form von Hydraten oder Solvaten.

7. Verfahren zur Herstellung von Verbindungen der Formel (I) nach einem der Ansprüche 1 bis 5, worin R₃ von einer -CO-NR₈R₉-Gruppe verschieden ist, **dadurch gekennzeichnet, daß** man eine Verbindung der Formel (II), wobei R für eine elektrophile Gruppe steht und Pg für eine Schutzgruppe steht, mit einem Epoxid der Formel (III), wobei R₁ und R₂ die in einem der Ansprüche 1 bis 3 angegebene Bedeutung besitzen: 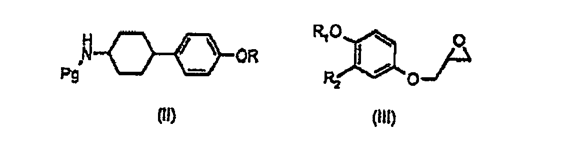 zu einer Verbindung der Formel (IV): 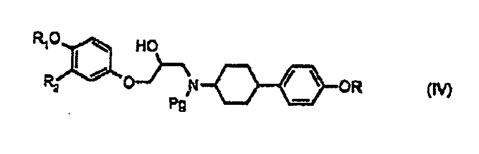 umsetzt,
welche man in eine Verbindung der Formel (V) umwandelt, worin R₃ die in einem der Ansprüche 1 bis 4 angegebene Bedeutung besitzt, aber von einer -CO-NR₈R₉-Gruppe verschieden ist: 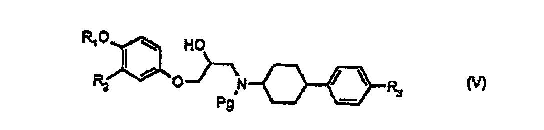 von der man danach die Schutzgruppen abspaltet.

8. Verfahren zur Herstellung von Verbindungen der Formel (I) nach einem der Ansprüche 1 bis 6, worin R₃ für eine -CO-NR₈R₉-Gruppe steht, **dadurch gekennzeichnet, daß** man eine Verbindung der Formel (XII): 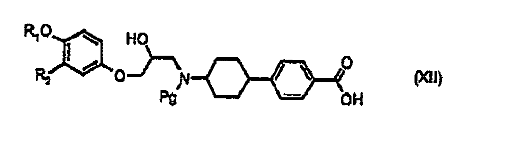 wobei R₁ und R₂ die in einem der Ansprüche 1 bis 4 angegebene Bedeutung besitzen und Pg für eine Schutzgruppe steht, mit einem Amin der Formel HNR₈R₉, worin R₈ und R₉ die in einem der Ansprüche 1 bis 6 angegebene Bedeutung besitzen, umsetzt und dann von dem so erhaltenen Produkt die Schutzgruppen abspaltet.

9. Arzneimittel, **dadurch gekennzeichnet, daß** es eine Verbindung der Formel (I) nach einem der Ansprüche 1 bis 6 oder ein Additionssalz dieser Verbindung mit einer pharmazeutisch unbedenklichen Säure oder auch ein Hydrat oder ein Solvat der Verbindung der Formel (I) enthält.

10. Pharmazeutische Zusammensetzung, **dadurch gekennzeichnet, daß** sie eine Verbindung der Formel (I) nach einem der Ansprüche 1 bis 6 oder ein pharmazeutisch unbedenkliches Salz, ein Hydrat oder ein Solvat dieser Verbindung und mindestens einen pharmazeutisch unbedenklichen Trägerstoff enthält.

11. Verwendung einer Verbindung der Formel (I) nach einem der Ansprüche 1 bis 6 zur Herstellung eines Arzneimittels zur Behandlung von Magen-DarmErkrankungen, als Modulatoren der Darmmotorik, als Lipolytika, Antiadiposita, Antidiabetika, Antiglaukomata, Vernarbungsmittel, als Uteruskontraktionshemmer, als Tocolytika zur Prävention oder Verlangsamung von vorzeitigen Wehen, zur Behandlung und/oder Prophylaxe von Dysmenorrhoe, als Psychotropika oder Antidepressiva sowie zur Behandlung von Harninkontinenz.

## Patentansprüche (Patentansprüche für folgende(n) Vertragsstaat(en): AT, BE, CH, CY, DE, DK, ES, FI, FR, GB, GR, IE, IT, LI, LU, MC, NL, PT,SE, TR)

1. Verbindungen der Formel (I) 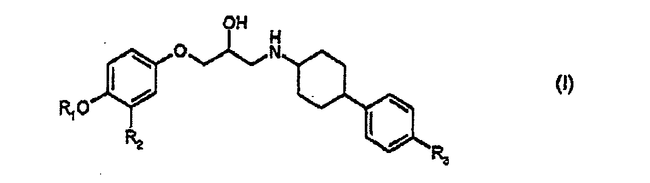 worin:
R₁ für ein Wasserstoffatom oder eine (C₁-C₄) -Alkylgruppe, eine -CO-(C₁-C₄)-Alkylgruppe, eine (C₁-C₄)-Alkylphenylgruppe oder eine -CO-Phenylgruppe steht, wobei das Phenyl gegebenenfalls durch eine bis drei unabhängig voneinander unter Halogenatomen, (C₁-C₄)-Alkylgruppen und (C₁-C₄)-Alkoxygruppen ausgewählte Gruppen substituiert ist;
R₂ unter den folgenden Gruppen ausgewählt ist:
. ein Wasserstoffatom,
. ein Halogenatom,
. eine -S(O)_{z}-(C₁-C₄) -Alkylgruppe, wobei z gleich 0, 1 oder 2 ist,
. eine -NHSO₂-(C₁-C₄)-Alkylgruppe,
. eine -NHSO₂-Phenylgruppe oder
. eine -NHSO₂-(C₁-C₄)-Alkylphenylgruppe,
wobei das Phenyl gegebenenfalls durch eine bis drei unabhängig voneinander unter Halogenatomen, (C₁-C₄)-Alkylgruppen und (C₁-C₄) -Alkoxygruppen ausgewählte Gruppen substituiert ist; und
R₃ unter den folgenden Gruppen ausgewählt ist:
• eine -X-R₄-Gruppe, worin X für eine Bindung, ein Sauerstoffatom oder eine -CH₂-Gruppe steht und R₄ für ein Wasserstoffatom oder eine Gruppe der Formel -CR₅R₆-COOR₇, wobei R₅, R₆ und R₇ unabhängig voneinander ein Wasserstoffatom oder eine (C₁-C₄)-Alkylgruppe bedeuten, steht, wobei R₄ jedoch nicht für ein Wasserstoffatom steht, wenn X für eine Bindung steht,
• eine Phenylgruppe, die gegebenenfalls durch eine bis drei unabhängig voneinander unter Halogenatomen, (C₁-C₄) -Alkylgruppen und (C₁-C₄) - Alkoxygruppen ausgewählte Gruppen substituiert oder mit einer Dioxolangruppe anelliert ist,
• eine -CO-NR₈R₉-Gruppe, worin R₈ für ein Wasserstoffatom, eine (C₁-C₉)-Alkylgruppe oder eine (C₁-C₄)-Alkoxy-(C₁-C₄)-alkylgruppe steht und R₉ unter den folgenden Gruppen ausgewählt ist:
- eine (C₁-C₄) -Alkoxy- (C₁-C₄) -alkylgruppe,
- eine Gruppe der Formel -(CH₂)ₙ-A, wobei n gleich 0, 1, 2, 3 oder 4 ist und A für eine Indolyl-, Fluoren- oder Phenylgruppe steht, wobei die Phenylgruppe durch eine bis drei unabhängig voneinander unter Halogenatomen und Hydroxy- oder (C₁-C₄)-Alkylgruppen ausgewählte Gruppen substituiert ist,
- eine -NH-Phenylgruppe, wobei die Phenylgruppe gegebenenfalls durch eine bis drei unabhängig voneinander unter Halogenatomen und Hydroxy- oder (C₁-C₄) -Alkylgruppen ausgewählte Gruppen substituiert ist, oder
- eine Gruppe der Formel -CH(R₁₀)-(CH₂)ₙ-COOR₁₁, wobei n gleich 0, 1, 2 oder 3 ist, R₁₁ für ein Wasserstoffatom oder eine (C₁-C₄)-Alkylgruppe steht und R₁₀ für:
. ein Wasserstoffatom,
. eine (C₁-C₄)-Alkylgruppe,
. eine -COOR₁₂-Gruppe, wobei R₁₂ ein Wasserstoffatom oder eine (C₁-C₉)-Alkylgruppe bedeutet, oder
. eine -CH₂-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist,
steht;
in Form von Basen oder Säureadditionssalzen und in Form von Hydraten oder Solvaten unter Ausschluß der Verbindungen, für die R₃ für eine X-R₄-Gruppe steht, wobei X eine -CH₂-Gruppe bedeutet und R₄ Wasserstoff bedeutet, wenn R₁ für ein Wasserstoffatom, eine (C₁-C₄)-Alkylgruppe, eine -CO- (C₁-C₄) - Alkylgruppe oder eine -CO-Phenylgruppe steht, wobei das Phenyl gegebenenfalls durch eine unter einem Halogenatom, einer (C₁-C₄)-Alkylgruppe und einer (C₁-C₄)-Alkoxygruppe ausgewählte Gruppe substituiert ist; und
R₂ unter den folgenden Gruppen ausgewählt ist:
. ein Wasserstoffatom,
. ein Halogenatom,
. eine -S(O)_{z}-(C₁-C₄)-Alkylgruppe, wobei z gleich 0, 1 oder 2 ist,
. eine -NHSO₂-(C₁-C₄)-Alkylgruppe oder
. eine -NHSO₂-Phenylgruppe,
wobei das Phenyl gegebenenfalls durch eine
unter einem Halogenatom, einer (C₁-C₄)-Alkylgruppe und einer (C₁-C₄)-Alkoxygruppe ausgewählte Gruppe substituiert ist.

2. Verbindungen nach Anspruch 1, **dadurch gekennzeichnet, daß**:
R₃ unter den folgenden Gruppen ausgewählt ist:
• eine -X-R₄-Gruppe, worin X für eine Bindung oder eine -CH₂-Gruppe steht und R₄ für eine Gruppe der Formel -CR₅R₆-COOR₇ steht; oder X für ein Sauerstoffatom steht und R₄ für ein Wasserstoffatom oder eine Gruppe der Formel -CR₅R₆-CO0R₇ steht, wobei R₅, R₆ und R₇ unabhängig voneinander ein Wasserstoffatom oder eine (C₁-C₄)-Alkylgruppe bedeuten,
• eine Phenylgruppe, die gegebenenfalls durch eine bis drei unabhängig voneinander unter Halogenatomen, (C₁-C₄)-Alkylgruppen und (C₁-C₄) - Alkoxygruppen ausgewählte Gruppen substituiert oder mit einer Dioxolangruppe anelliert ist, oder
• eine -CO-NR₈R₉-Gruppe, worin R₈ für ein Wasserstoffatom, eine (C₁-C₄)-Alkylgruppe oder eine (C₁-C₄)-Alkoxy-(C₁-C₄)-alkylgruppe steht und R₉ unter den folgenden Gruppen ausgewählt ist:
- eine (C₁-C₄)-Alkoxy-(C₁-C₄)-alkylgruppe,
- eine Gruppe der Formel -(CH₂)ₙ-A, wobei n gleich 0, 1, 2, 3 oder 4 ist und A für eine Indolyl-, Fluoren- oder Phenylgruppe steht, wobei die Phenylgruppe durch eine bis drei unabhängig voneinander unter Halogenatomen und Hydroxy- oder (C₁-C₄)-Alkylgruppen ausgewählte Gruppen substituiert ist,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH(R₁₀)-(CH₂)ₙ-COOR₁₁, wobei n gleich 0, 1, 2 oder 3 ist, R₁₁ für ein Wasserstoffatom oder eine (C₁-C₉)-Alkylgruppe steht und R₁₀ für:
. ein Wasserstoffatom,
. eine (C₁-C₄) -Alkylgruppe,
. eine -COOR₁₂-Gruppe, wobei R₁₂ ein Wasserstoffatom oder eine (C₁-C₄)-Alkylgruppe bedeutet, oder
. eine -CH₂-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist,
steht;
in Form von Basen oder Säureadditionssalzen und in Form von Hydraten oder Solvaten.

3. Verbindungen der Formel (I) nach Anspruch 1 oder 2, **dadurch gekennzeichnet, daß** R₁ für ein Wasserstoffatom steht,
in Form von Basen oder Säureadditionssalzen und in Form von Hydraten oder Solvaten.

4. Verbindungen der Formel (I) nach Anspruch 1, 2 oder 3, **dadurch gekennzeichnet, daß** R₂ für eine -S(O)₂- (C₁-C₄) -Alkyl- oder -NHSO₂- (C₁-C₄) -Alkylgruppe steht,
in Form von Basen oder Säureadditionssalzen und in Form von Hydraten oder Solvaten.

5. Verbindungen nach einem der Ansprüche 1 bis 4, **dadurch gekennzeichnet, daß** R₃ unter den folgenden Gruppen ausgewählt ist:
• eine -X-R₄-Gruppe, worin X und R₄ die in Anspruch 1 oder 2 angegebene Bedeutung besitzen,
• eine Phenylgruppe, die gegebenenfalls durch eine bis drei unabhängig voneinander unter Halogenatomen, (C₁-C₄)-Alkylgruppen und (C₁-C₄)-Alkoxygruppen ausgewählte Gruppen substituiert oder mit einer Dioxolangruppe anelliert ist,
• eine -CO-NR₈R₉-Gruppe, worin R₈ die in Anspruch 1 angegebene Bedeutung besitzt und R₉ unter den folgenden Gruppen ausgewählt ist:
- eine (C₁-C₉)-Alkoxy-(C₁-C₄)-alkylgruppe,
- eine Gruppe der Formel -(CH₂)ₙ-A, wobei n gleich 0, 1, 2, 3 oder 4 ist und A für eine Indolylgruppe, eine Fluorengruppe oder eine Phenylgruppe, die durch eine bis drei unabhängig voneinander unter Halogenatomen und Hydroxy- oder (C₁-C₄)-Alkylgruppen ausgewählte Gruppen substituiert ist, steht,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH (R₁₀)- (CH₂)ₙ-COOR₁₁, wobei n gleich 0, 1, 2 oder 3 ist und R₁₀ und R₁₁ die in Anspruch 1 angegebene Bedeutung besitzen;
in Form von Basen oder Säureadditionssalzen und in Form von Hydraten oder Solvaten.

6. Verbindungen nach Anpruch 5, **dadurch gekennzeichnet, daß** R₃ für eine -CO-NHR₉-Gruppe steht, worin R₉ unter den folgenden Gruppen ausgewählt ist:
- eine Gruppe der Formel -(CH₂)ₙ-A, wobei n gleich 0, 1, 2, 3 oder 4 ist und A für eine Indolylgruppe, eine Fluorengruppe oder eine Phenylgruppe, die durch eine bis drei unabhängig voneinander unter Halogenatomen und Hydroxy- oder (C₁-C₄)-Alkylgruppen ausgewählte Gruppen substituiert ist, steht,
- eine -NH-Phenylgruppe, wobei die Phenylgruppe gegebenenfalls durch eine bis drei unabhängig voneinander unter Halogenatomen und Hydroxy- oder (C₁-C₄)-Alkylgruppen ausgewählte Gruppen substituiert ist, oder
- eine Gruppe der Formel -CH (R₁₀) - (CH₂)ₙ-COOR₁₁, wobei n gleich 0, 1, 2 oder 3 ist und R₁₀ und R₁₁ die in Anspruch 1 angegebene Bedeutung besitzen;
in Form von Basen oder Säureadditionssalzen und in Form von Hydraten oder Solvaten.

7. Verfahren zur Herstellung von Verbindungen der Formel (I) nach einem der Ansprüche 1 bis 4, worin R₃ von einer -CO-NR₈R₉-Gruppe verschieden ist, **dadurch gekennzeichnet, daß** man eine Verbindung der Formel (II), wobei R für eine elektrophile Gruppe steht und Pg für eine Schutzgruppe steht, mit einem Epoxid der Formel (III), wobei R₁ und R₂ die in einem der Ansprüche 1 bis 4 angegebene Bedeutung besitzen: 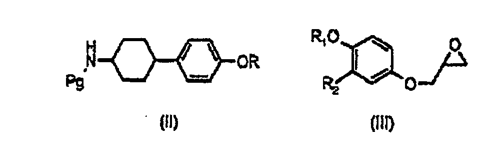 zu einer Verbindung der Formel (IV): 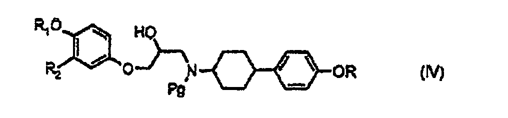 umsetzt,
welche man in eine Verbindung der Formel (V) umwandelt, worin R₃ die in einem der Ansprüche 1 bis 5 angegebene Bedeutung besitzt, aber von einer -CO-NR₈R₉-Gruppe verschieden ist: 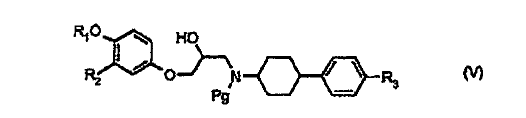 von der man danach die Schutzgruppen abspaltet.

8. Verfahren zur Herstellung von Verbindungen der Formel (I) nach einem der Ansprüche 1 bis 6, worin R₃ für eine -CO-NR₈R₉-Gruppe steht, **dadurch gekennzeichnet, daß** man eine Verbindung der Formel (XII): 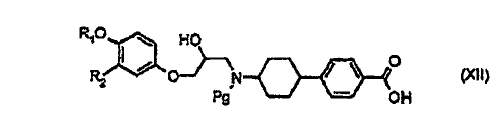 wobei R₁ und R₂ die in einem der Ansprüche 1 bis 4 angegebene Bedeutung besitzen und Pg für eine Schutzgruppe steht, mit einem Amin der Formel HNR₈R₉, worin R₈ und R₉ die in einem der Ansprüche 1 bis 6 angegebene Bedeutung besitzen, umsetzt und dann von dem so erhaltenen Produkt die Schutzgruppen abspaltet.

9. Arzneimittel, **dadurch gekennzeichnet, daß** es eine Verbindung der Formel (I) nach einem der Ansprüche 1 bis 6 oder ein Additionssalz dieser Verbindung mit einer pharmazeutisch unbedenklichen Säure oder auch ein Hydrat oder ein Solvat der Verbindung der Formel (I) enthält.

10. Pharmazeutische Zusammensetzung, **dadurch gekennzeichnet, daß** sie eine Verbindung der Formel (I) nach einem der Ansprüche 1 bis 6 oder ein pharmazeutisch unbedenkliches Salz, ein Hydrat oder ein Solvat dieser Verbindung und mindestens einen pharmazeutisch unbedenklichen Trägerstoff enthält.

11. Verwendung einer Verbindung der Formel (I) nach einem der Ansprüche 1 bis 6 zur Herstellung eines Arzneimittels zur Behandlung von Magen-DarmErkrankungen, als Modulatoren der Darmmotorik, als Lipolytika, Antiadiposita, Antidiabetika, Antiglaukomata, Vernarbungsmittel, als Uteruskontraktionshemmer, als Tocolytika zur Prävention oder Verlangsamung von vorzeitigen Wehen, zur Behandlung und/oder Prophylaxe von Dysmenorrhoe, als Psychotropika oder Antidepressiva sowie zur Behandlung von Harninkontinenz.
